# EUROPÄISCHE PATENTSCHRIFT

(11) **EP 0 923 546 B1**
(45) Veröffentlichungstag und Bekanntmachung des Hinweises auf die Patenterteilung: **26.11.2003**
(21) Anmeldenummer: 97934481.9
(22) Anmeldetag: 17.07.1997
(51) Int. Cl.: C07D 207/32, C07D 207/34, C07D 209/42, A61K 31/40, A61K 31/405

(54) **ACYLPYRROLDICARBONSÄUREN UND ACYLINDOLDICARBONSÄUREN SOWIE IHRE DERIVATE ALS HEMMSTOFFE DER CYTOSOLISCHEN PHOSPHOLIPASE A2**
ACYLPYRROLDICARBOXYLIC ACIDS AND ACYLINDOLDICARBOXYLIC ACIDS AND THEIR DERIVATIVES AND INHIBITORS OF THE CYTOSOLIC PHOSPHOLIPASE A2
ACIDES ACYLPYRROLDICARBOXYLIQUES ET ACIDES ACYLINDOLDICARBOXYLIQUES ET LEURS DERIVES UTILISES EN TANT QU'INHIBITEURS DE LA PHOSPHOLIPASE A2 CYTOSOLIQUE

(30) Priorität: 01.08.1996 DE 19631102
(43) Veröffentlichungstag der Anmeldung: 23.06.1999
(73) Patentinhaber: Merckle GmbH, D-89143 Blaubeuren (DE)
(72) Erfinder: LEHR, Matthias, D-81377 München (DE)
(74) Vertreter: Best, Michael, Dr.
(86) Internationale Anmeldenummer: EP9703842
(87) Internationale Veröffentlichungsnummer: WO98005637

(56) Entgegenhaltungen:
- EP-A- 0 377 539
- EP-A- 0 397 175
- EP-A- 0 620 214
- WO-A-91/06537
- WO-A-92/02132
- WO-A-95/13266
- WO-A-96/03376
- US-A- 3 624 103
- SCHEVITZ,R.W. ET AL.: "Structure-based design of the first potent and selective inhibitor of human non-pancreatic secretory phospholipase A2 " NAT.STRUCT.BIOL., Bd. 2, Nr. 6, 1995, WASHINGTON, Seiten 458-465, XP002046508
- DILLARD,R.D. ET AL.: "Indole Inhibitors of Human Nonpancreatic Secretory Phospholipase A2. 1. Indole-3-acetamides" J.MED.CHEM., Bd. 39, Nr. 26, 20.Dezember 1996, WASHINGTON, Seiten 5119-5136, XP002046054
- LEHR,M.: "3-(Octadecanoylaminomethyl)indole-2-carbo xylic Acid Derivatives and 1-Methyl-3-octadecanoylindole-2-carboxylic Acid as Inhibitors of Cytosolic Phospholipase A2 " ARCH.PHARM., Bd. 329, August 1996, WEINHEIM, Seiten 386-392, XP002046509
- DILLARD,R.D. ET AL.: "Indole Inhibitors of Human Nonpancreatic Secretory Phospholipase A2. 2. Indole-3-acetamides with Additional Functionality " J.MED.CHEM., Bd. 39, Nr. 26, 20.Dezember 1996, WASHINGTON, Seiten 5137-5158, XP002046055
- LEHR,M.: "Synthesis, Biological Evaluation and Structure-Activity Relationships of 3-Acylindole-2-carboxylic Acids as Inhibitors of the Cytosolic Phospholipase A2 " J.MED.CHEM., Bd. 40, Nr. 17, August 1997, WASHINGTON, Seiten 2694-2705, XP002045903

## Beschreibung

Die vorliegende Erfindung betrifft neuartige Acylpyrroldicarbonsäuren und Acylindoldicarbonsäuren sowie deren Derivate, welche das Enzym Phospholipase A₂ hemmen. Diese Verbindungen sind geeignet als Arzneimittel zur Prävention und zur Behandlung von Erkrankungen, die durch eine erhöhte Aktivität dieses Enzyms verursacht bzw. mitverursacht werden, wie z.B. Entzündungen, Schmerz, Fieber, Allergien, Asthma, Psoriasis und Endotoxinschock. Die Erfindung betrifft ferner Methoden zur Synthese dieser Verbindungen sowie pharmazeutische Mittel, die diese Verbindungen enthalten.

Es ist bekannt, daß die Phospholipase A₂ hydrolytisch die Esterbindung in 2-Position von Membranphospholipiden spaltet, wobei freie Fettsäuren, hauptsächlich Arachidonsäure, und lyso-Phospholipide entstehen.

Die freigesetzte Arachidonsäure wird über den Cyclooxygenase-Weg zu den Prostaglandinen und Thromboxanen sowie über die Lipoxygenase-Wege zu den Leukotrienen und anderen hydroxylierten Fettsäuren metabolisiert. Die Prostaglandine sind an der Enstehung des Schmerzes und des Fiebers sowie an entzündlichen Reaktionen wesentlich beteiligt. Leukotriene sind wichtige Mediatoren bei Entzündungsprozessen und bei anaphylaktischen und allergischen Vorgängen (Forth et al., Allgemeine und Spezielle Pharmakologie und Toxikologie BI Wissenschaftsverlag Mannheim, Wien, Zürich, 1987).

Die durch die Phospholipase A₂ gebildeten lyso-Phospholipide besitzen zellschädigende Eigenschaften. Lyso-Phosphatidylserin führt zur Freisetzung des an allergischen Prozessen beteiligten Histamins (Moreno et al., Agents Actions 1992, 36, 258). Lyso-Phosphatidylcholin wird darüber hinaus zum plättchenaktivierenden Faktor (PAF) metabolisiert, der ebenfalls ein wichtiger Mediator z.B. bei Entzündungen ist.

Da die Phospholipase A₂ das Schlüsselenzym für die Bildung der genannten pathophysiologisch bedeutsamen Mediatoren darstellt, lassen sich durch eine Hemmung des Enzyms diese Mediatorwirkungen ausschalten.

Es sind bereits einige Pyrrolderivate als Antiphlogistika und Analgetika bekannt. Die Wirkung des bereits als Arzneimittel zugelassenen Stoffes Tolmetin (5-(4-Methylbenzoyl)-pyrrol-2-ylessigsäure) (U. Ficke et al. Neue Arzeimittel 1993, Wissenschaftliche Verlagsgesellschaft, Stuttgart 1994, S. 20 ff) und der in der deutschen Offenlegungsschrift 3,415,321 offenbarten Benzoylpyrrol-alkansäuren beruht auf einer Cyclooxygenase-Hemmung. Eine Hemmung der Cyclooxygenase hat zur Folge, daß dem Lipoxygenasestoffwechsel vermehrt Arachidonsäure zur Verfügung steht, die in einem vorgelagerten Schritt in einer Phospholipase A₂ katalysierten Reaktion synthetisiert wird. Dadurch werden bestimmte Symptome der Entzündung, die durch Lipoxygenase-abhängige Arachidonsäurederivate hervorgerufen werden, noch verstärkt. Die deutsche OS 2,302,669 offenbart 1-Methyl-5-(3-phenylacryloyl)-pyrrol-2-yl-ameisensäure als eine Verbindung mit analgetischer wirkung bei Mäusen.

Weiterhin sind einige verbindungen als Phospholipase A₂-Inhibitoren bekannt. WO 88-06,885 offenbart Aminoalkylamide und EP-A-377 539 offenbart 4-Aryloyl-pyrrol-2-yl-ameisensäuren mit Phospholipase A₂-hemmender Wirkung.

WO 91/06537 offenbart substituierte Inden-, Indol-, Pyranoindol- und Tetrahydrocarbazol-alkansäurederivate, die Lipoxygenase-hemmende, Phospholipase A₂-hemmende und Leukotrien-antagonistische Wirkung besitzen und als entzündungshemmende, antiallergische und zytotoxische Mittel geeignet sind.

In der Druckschrift Schewitz et al. Nature Structual Biology 1995, Vol. 2, Nr. 6, 458-465 wird ein wirksamer und selektiver Inhibitor der "human non-pancreatic secretory" Phospholipase A₂ (hnps-PLA₂) offenbart.

Die EP-A-0 620 214 offenbart eine Klasse von 1H-Indol-3-essigsäurehydraziden, die durch human-sPLA₂ vermittelte Freisetzung von Arachidonsäure hemmt. Ferner wird in der Druckschrift ein Verfahren zur Vorbeugung und Behandlung von septischen Schock unter Verwendung von 1H-Indol-3-essigsäurehydraziden offenbart.

Die WO 96/03376 offenbart 1H-Indol-1-glyoxylamide, 1H-Indol-1-acetamide und 1H-Indol-1-hydrazide als Hemmstoffe für die sPLA₂ vermittelte Freisetzung von Fettsäuren bei Zuständen wie septischem Schock.

Die US 3,624,103 offenbart 2-Methyl-5-methoxy-3-indolacetohydroxamsäure-Derivate, die wirkungsvolle entzündungshemmende, fiebersenkende und analgetische Mittel darstellen.

Die EP-A-0 397 175 offenbart, dass bestimmte substituierte pyrrolverbindungen potente Cyclooxygenase- und/oder Lipoxygenase-hemmer sind und daher zur Prävention von allergisch induzierten Erkrankungen und zur Behandlung von Erkrankungen des rheumatischen Formenkreises brauchbar sind.

Die WO 92/03132 offenbart, dass bestimmte substituierte Indolylverbindungen wirkungsvolle Hemmstoffe der Leukotrien-Biosynthese sind und daher zur Behandlung oder Verbesserung von Zuständen entzündlicher Krankheiten geeignet sind, in welchen Leukotriene eine Rolle spielen.

Die DE-A-4325204 offenbart, dass bestimmte Acylpyrrolalkansäuren und deren Derivate potente Phospholipase A₂-Inhibitoren sind und daher zur Behandlung von Erkrankungen, die durch eine erhöhte Aktivität dieses Enzyms verursacht, bzw. mitverursacht werden, wie zum Beispiel Entzündungen, Allergien, Asthma, Psoriasis und Endotoxinschock, brauchbar sind.

Indol-2-alkansäuren als Analgetika mit Prostaglandin- und Thromboxan-hemmender Wirkung werden in dem US-Patent Nr. 5,081,145 offenbart. US-Patent Nr. 5,132,319 beschreibt 1-(Hydroxylaminoalkyl)indolderivate, die die Leukotrien-Biosynthese inhibieren. Hierdurch besitzen diese Verbindungen eine schmerz- und entzündungshemmende Wirkung. Die in der EP-A-535 923 offenbarten (Azaarylmethoxy)indole inhibieren ebenfalls die Leukotrien-Biosynthese.

Daß bestimmte Acylpyrrolalkansäuren und Indol-2-alkansäuren sowie ihre Derivate Phospholipase A₂ hemmen können, ist auch aus der WO 95/13266 bekannt. Die dort offenba rten Acylpyrrolalkansäuren und Indol-2-alkansäuren sind zwar potente Phospholipase-A₂-Inhibitoren, jedoch besteht auf dem Fachgebiet das Bedürfnis nach neuen Verbindungen, die eine noch verbesserte Hemmwirkung und/oder eine geringere Zytotoxizität aufweisen.

Es ist daher die Aufgabe der vorliegenden Erfindung, Antiphlogistika und Analgetika zur Verfügung zu stellen, die gegenüber den aus dem Stand der Technik bekannten verbindungen eine verbesserte Hemmwirkung und/oder eine geringere Zytotoxizität aufweisen. Während die antiphlogistischen und analgetischen Wirkungen der derzeit therapeutisch verfügbaren nichtsteroidalen Antiphlogistika auf der Hemmung der Prostaglandinbildung infolge einer Hemmung des Enzyms Cyclooxygenase beruhen, sollen die beanspruchten Substanzen, wie die in der WO 95/13266 offenbarten Verbindungen, das Enzym Phospholipase A₂ inhibieren. Dadurch wird nicht nur die Biosynthese der an entzündlichen Prozessen und am Schmerzgeschehen beteiligten prostaglandine, sondern auch die Bildung der Leukotriene, des plättchenaktivierenden Faktors und der lyso-Phospholipide unterbunden.

Es wurde nun unerwartet gefunden, daß Pyrrolcarbonsäurederivate und Indolcarbonsäurederivate mit bestimmten Substituentenkombinationen eine verbesserte Hemmwirkung bzw. eine geringere Zytotoxizität aufweisen als die bekannten Derivate und daher besser als diese zur Prävention und/oder zur Behandlung von Erkrankungen, die durch eine erhöhte Aktivität des Enzyms Phospholipase A₂ verursacht bzw. mitverursacht werden, wie z.B. Entzündungen, Allergien, Asthma, Psoriasis und Endotoxinschock, brauchbar sind.

Es ist bekannt, daß es mehrere verschiedene Phospholipasen A₂ gibt (Connolly und Robinson, Drug News & Perspectives 1993, 6, 584-590). Das Schlüsselenzym bei der Biosynthese der genannten pathophysiologisch bedeutsamen Lipidmediatoren ist die sogenannte cytosolische Phospholipase A₂ (cPLA₂) (Clark et al, J. Lipid Mediators Cell Signalling 1995, 12, 83-117). Die erfindungsgemäßen Verbindungen hemmen insbesondere diese cPLA₂.

Gegenstand der vorliegenden Erfindung sind somit substituierte Pyrrolverbindungen und substituierte Indolverbindungen der allgemeinen Formeln I und II: 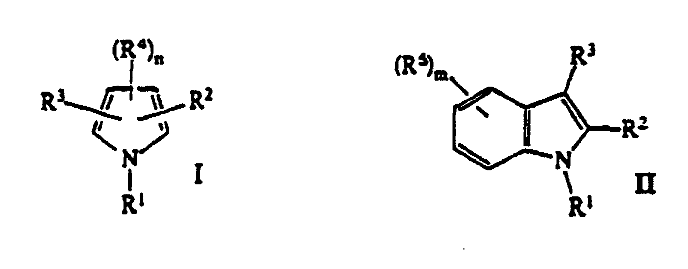 worin
R¹ ein Rest -Y¹-Ar-Y²-Y³ ist, wobei Y¹ ein C₁-C₁₂-Alkyl-, C₂-C₁₂-Alkenyl-, C₁-C₁₂-Alkyloxy- oder C₂-C₁₂-Alkenyloxy-Rest ist, der gegebenenfalls durch ein oder mehrere Sauerstoffatome unterbrochen sein kann, Ar eine Arylgruppe ist, die gegebenenfalls mit 1 bis 3 Substituenten ausgewählt aus der Gruppe R⁶, R⁷ und R⁸ substituiert sein kann, Y² ein C₁-C₁₂-Alkyl-, C₂-C₁₂-Alkenyl-, C₁-C₁₂-Alkyloxy- oder C₂-C₁₂-Alkenyloxy-Rest ist, der gegebenenfalls durch ein oder mehrere Sauerstoffatome unterbrochen sein kann, und Y³ für - COOR¹⁷, -CONR¹⁷R¹⁷, -CONHCOR¹⁹, -CONHS(O)₂R¹⁹, - CONHNHS(O)₂R¹⁹ bzw. -Tz steht, wobei Tz 1H- oder 2H-Tetrazol-5-yl bedeutet;
R² für -COOR¹⁷, -Y⁴-COOR¹⁷, -CONR¹⁷R¹⁷, -Y⁴-CONR¹⁷R¹⁷, - CONHCOR¹⁹, -Y⁴-CONHCOR¹⁹, -CONHS(O)₂R¹⁹, -Y⁴-CONHS(O)₂R¹⁹, -CONHNHS(O)₂R¹⁹, -Y⁴-CONHNHS(O)₂R¹⁹, -Tz bzw. -Y⁴-Tz steht, wobei Y⁴ eine C₁-C₈-Alkyl- oder C₂-C₈-Alkenylgruppe ist, die gegebenenfalls durch ein Sauerstoffatom unterbrochen sein kann und Tz 1H- oder 2H-Tetrazol-5-yl bedeutet;
R³ für -CO-R⁹ steht, wobei R⁹ für -Y⁵, -Aryl oder -Y⁵-Aryl steht, wobei Y⁵ eine C₁-C₁₉-Alkyl- oder C₂-C₁₉-Alkenyl- oder -Alkinyl-Gruppe ist, die gegebenenfalls durch ein oder mehrere Sauerstoffatome unterbrochen sein kann, und Aryl eine gegebenenfalls mit 1 bis 3 Substituenten ausgewählt aus der Gruppe R¹⁰, R¹¹ und R¹² substituierte Arylgruppe ist;
jeder Rest R⁴ unabhängig voneinander für ein Wasserstoffatom, ein Halogenatom, -CF₃, -Y⁶, -Aryl oder -Y⁶-Aryl steht, wobei Y⁶ eine C₁-C₈-Alkyl- oder C₂-C₈-Alkenyl- oder Alkinyl-Gruppe ist, die gegebenenfalls durch ein oder mehrere Sauerstoffatome unterbrochen sein kann, und Aryl eine gegebenenfalls mit 1 bis 3 Substituenten ausgewählt aus der Gruppe R¹³, R¹⁴ und R¹⁵ substituierte Arylgruppe ist und n die Zahl 2 ist; und wobei zwei Reste Y⁶, sofern es sich um zwei benachbart stehende Alkylreste handelt, zusammen mit dem Kohlenstoffatom, an das sie gebunden sind, einen 5-8-gliedrigen Ring bilden können, der gegebenenfalls mit 1 bis 2 C₁-C₄-Alkylgruppen substituiert sein kann;
jeder Rest R⁵ unabhängig voneinander für ein Wasserstoffatom oder R¹⁶ steht und m die Zahl 4 ist;
R⁶, R⁷, R⁸, R¹⁰, R¹¹, R¹², R¹³, R¹⁴, R¹⁵ und R¹⁶ unabhängig voneinander ausgewählt sind aus:
(1) C₁-C₂₀-Alkylgruppe, die gegebenenfalls durch ein Sauerstoffheteroatom unterbrochen sein kann;
(2) C₂₋₂₀-Alkenylgruppe, die gegebenenfalls durch ein Sauerstoffheteroatom unterbrochen sein kann;
(3) C₂₋₂₀-Alkinylgruppe, die gegebenenfalls durch ein Sauerstoffheteroatom unterbrochen sein kann;
(4) Halogen;
(5) -CF₃;
(6) Perhalo-C₁-C₆-Alkenyl;
(7) -CN;
(8) -NO₂;
(9) -OR¹⁷;
(10) -SR¹⁷;
(11) -COOR¹⁷;
(12) -COR¹⁸;
(13) -COCH₂OH;
(14) -NHCOR¹⁷;
(15) -NR¹⁷R¹⁷;
(16) -NHS(O)₂R¹⁷;
(17) -SOR¹⁷;
(18) -S(O)₂R¹⁷;
(19) -CONR¹⁷R¹⁷;
(20) -SO₂NR¹⁷R¹⁷;
(21) -OOCR¹⁸;
(22) -OOCNR¹⁷R¹⁷;
(23) -OOCOR¹⁷;
(24) -(CH₂)ᵣOR²³;
(25) -(CH₂)ᵣSR²³;
(26) -(CH₂)ᵣNHR²³;
(27) -(CH₂)ₛR²⁰;
R¹⁷ jeweils unabhängig voneinander Wasserstoff, eine C₁₋C₂₀-Alkyl- bzw. C₂₋C₁₉-Alkenyl- oder -Alkinylgruppe, die gegebenenfalls durch ein Sauerstoffheteroatom unterbrochen sein kann, oder -(CH₂)ₜR²⁰ bedeutet;
R¹⁸ jeweils unabhängig voneinander R¹⁷, -CF₃, - (CH₂)ᵤCOOH oder -(CH₂)ᵤCOOR²¹ bedeutet;
R¹⁹ jeweils unabhängig voneinander R¹⁷ oder -CF₃ bedeutet;
R²⁰ jeweils unabhängig voneinander Aryl, substituiert mit einer oder zwei R²²-Gruppen bedeutet;
R²¹ jeweils unabhängig voneinander C₁₋C₆-Alkyl, Benzyl oder Phenyl bedeutet;
R²² jeweils unabhängig voneinander Wasserstoff, Halogen, C₁₋C₁₂-Alkyl, C₁₋C₁₂-Alkoxy, C₁₋C₁₂-Alkylthio, C₁₋C₁₂-Alkylsulfonyl, C₁₋C₁₂-Alkylcarbonyl, -CF₃, -CN oder -NO₂ bedeutet;
R²³ jeweils unabhängig voneinander Wasserstoff oder -COR²¹ bedeutet;
r 1 bis 20 ist;
s und t jeweils unabhängig voneinander 0 bis 12 sind;
u 0 bis 4 ist;
sowie deren pharmazeutisch verträglichen Salze und Ester.

Erfindungsgemäß wurde gefunden, daß durch die spezielle Substitution des Stickstoffatoms eine verbesserte Hemmwirkung und/oder eine geringere Zytotoxizität der Verbindungen erhalten werden kann.

Die pharmazeutisch verträglichen Salze können Basenadditionssalze sein. Dazu zählen Salze der Verbindungen mit anorganischen Basen wie Alkalihydroxiden, Erdalkalihydroxiden oder mit organischen Basen, wie Mono-, Di- oder Triethanolamin. Auch Säureadditionssalze sind umfaßt.

Zu den pharmazeutisch verträglichen Estern der Verbindungen zählen insbesondere physiologisch leicht hydrolisierbare Ester, beispielsweise Alkyl-, Pivaloyloxymethyl-, Acetoxymethyl-, Phthalidyl-, Indanyl- und Methoxymethylenester.

Der hier verwendete Ausdruck "Alkyl" umfaßt geradkettige, verzweigte oder cyclische Alkylgruppen, wie Methyl, Ethyl, n-und iso-Propyl, n-, iso- oder t-Butyl, n-Pentyl, Neopentyl, n-Undecyl-, n-Dodecyl-, n-Pentadecyl, n-Hexadecyl, n-Heptadecyl, n-Octadecyl, Cyclopentyl, Cyclohexyl, Cyclododecyl etc.

Der Ausdruck "Alkenyl" umfaßt geradkettige, verzweigte oder cyclische Alkenylgruppen, wie Ethenyl, Propenyl, Butenyl, Decenyl, Heptadecenyl, Cyclopentenyl, Cyclohexenyl etc.

Der Ausdruck "Alkinyl" umfaßt geradkettige oder verzweigte Alkinylgruppen, wie Ethinyl, Propinyl, Butinyl, Decinyl, Heptadecinyl etc.

Der Ausdruck "Aryl" umfaßt aromatische Kohlenwasserstoffe mit 5 bis 14 Kohlenstoffatomen, die ein Heteroatom, wie Sauerstoff, Schwefel oder Stickstoff enthalten können. Insbesondere werden Phenyl-, Naphtyl- und Pyridylgruppen bevorzugt.

Der Ausdruck "Halogenatom" umfaßt Fluor-, Chlor-, Brom- oder Jodatom, wobei insbesondere Fluor- oder Chloratom bevorzugt ist.

Als Rest R² eignet sich für die vorliegende Erfindung insbesondere der entsprechende Rest der Ameisensäure, Essigsäure, 3-Propionsäure, 4-Buttersäure, 3-α-Methylpropionsäure, und 3-Acrylsäure. Bevorzugt werden Reste von Ameisensäure, Essigsäure, 3-Propionsäure, 3-α-Methylpropionsäure und 4-Buttersäure.

Als Rest R³ eignen sich insbesondere (C₁₋₁₉-Alkyl-, -Alkenyl- oder -Alkinyl)carbonylgruppen, die gegebenenfalls durch mehrere, insbesondere durch ein Sauerstoffatom, unterbrochen sein können. Weiterhin kann R³ mit einer Arylgruppe substituiert sein. Diese Arylgruppe kann gegebenenfalls einen oder mehrere, insbesondere einen oder zwei Substituenten enthalten. Erfindungsgemäß eignen sich als Substituenten Reste aus der Gruppe Halogenatom, Nitro-, Trifluormethyl-, C₄₋₁₂-Alkyl-, C₁₋₁₂-Alkoxy- und Hydroxygruppe. Besonders bevorzugt werden (C₇₋₁₇-Alkyl)carbonyl- und Aryl(C₁₋₁₇alkyl) carbonylgruppen.

Für den Substituenten R³ seien insbesondere die Gruppen Octanoyl, Nonanoyl, Decanoyl, Dodecanoyl, Hexadecanoyl und Octadecanoyl erwähnt.

Für die Reste R⁴ eignen sich insbesondere ein Wasserstoffatome eine C₁₋₈-Alkyl- oder C₂₋₈-Alkenyl- oder C₂₋₈-Alkinylgruppe, die gegebenenfalls durch ein oder mehrere Sauerstoffatome unterbrochen sein kann, eine gegebenenfalls substituierte Arylgruppe oder eine mit einem Arylrest substituierte C₁₋₈-Alkyl-, C₂₋₈-Alkenyl- oder C₂₋₈-Alkinylgruppe.

Sowohl die für R⁴ stehende Arylgruppe als auch die Arylgruppe Ar, die ein Substituent der Alkyl-, Alkenyl- oder Alkinylgruppe sein kann, kann substituiert sein. Bevorzugt sind dabei 1 oder 2 Substituenten ausgewählt aus C₁₋₄-Alkyl-, insbesondere Methyl-, C₁₋₄-Alkoxy-, insbesondere Methoxy-, Trifluormethyl-, Hydroxy-, Amino-, N,N-Di-C₁₋₄-alkylamino-, insbesondere N,N-Dimethylamino-, Amino-C₁₋₄-alkyl-, insbesondere Aminomethyl-, Cyano-, Amid-, N,N-Di-C₁₋₄alkylamid-, insbesondere N,N-Dimethylamid-, Carboxy-, C₁₋₄-Alkylsulfonyl-, insbesondere Methylsulfonylgruppe und Halogenatom.

Für die Reste R⁴ seien insbesondere Wasserstoff, Methyl, Ethyl, Propyl, Butyl, Pentyl, Neopentyl, Hexyl, Heptyl, Octyl, 3-Phenylpropyl, Phenyl und Benzyl erwähnt.

Besonders bevorzugt sind solche Verbindungen, in denen die Reste R⁴ jeweils unabhängig voneinander für ein Wasserstoffatom, einen C₁₋₅-Alkylrest, eine Benzyl- oder Phenylgruppe stehen.

Erfindungsgemäß werden für die Reste R² und R³ solche Reste bevorzugt, die, wenn vorhanden, gesättigte Alkylreste ohne Sauerstoffatome enthalten.

Die Erfindung umfaßt insbesondere Verbindungen der allgemeinen Formel I': 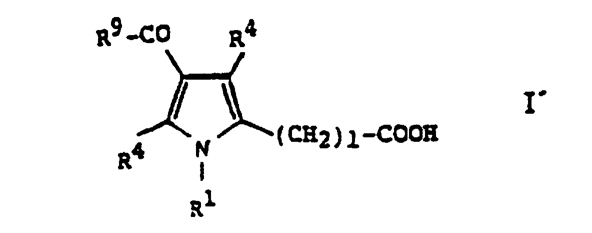 worin R⁹ bevorzugt für eine C₇₋₁₇-Alkyl- oder Aryl(C₁₋₁₇alkyl)gruppe mit gegebenenfalls sustituiertem Arylrest steht; und die Reste R⁴ jeweils unabhängig voneinander ausgewählt sind aus der Gruppe bestehend aus einem Wasserstoffatom, einer Methyl-, Phenyl- und Benzylgruppe; und l eine ganze zahl von 0 bis 3 ist.

Besonders bevorzugt sind Verbindungen der Formel I', in der l die Zahl 1 oder 2 ist, R⁹-CO- eine (C₇₋₁₇-Alkyl)carbonyloder Aryl(C₁₋₁₇-alkyl)carbonylgruppe und die Reste R⁴ jeweils unabhängig voneinander ein Wasserstoffatom oder eine Methyloder Benzylgruppe sind. Dabei sind insbesondere solche Verbindungen bevorzugt, bei denen l die Zahl 1 oder 2 ist, R⁹-CO eine (C₇₋₁₇-Alkyl)carbonylgruppe und die Reste R⁴ jeweils ein Wasserstoffatom oder eine Methylgruppe sind.

Weiterhin bevorzugt sind Verbindungen der Formel I', bei denen 1 die Zahl 1 oder 2 ist, R⁹-CO- eine Dodecanoylgruppe ist und die Reste R⁴ jeweils unabhängig voneinander ein Wasserstoffatom oder eine Methyl- oder Benzylgruppe sind.

Erfindungsgemäß besonders bevorzugt ist 3,5-Dimethyl-4-dodecanoyl-pyrrol-2-yl-essigsäure, die als Rest R¹ einen Rest -Y¹-Ar-Y²-Y³ trägt.

Der Rest Y¹ ist bevorzugt ein C₁-C₄-Alkylrest und besonders bevorzugt eine Methylengruppe. Der Rest Ar ist bevorzugt eine unsubstituierte Phenylengruppe und der Rest Y² ist bevorzugt ein C₁-C₆-Alkyl- oder C₂₋₆-Alkenylrest, besonders bevorzugt eine Ethenylen- oder Ethylengruppe. Der Rest Y² steht bevorzugt in meta- oder para-Stellung zu dem Rest Y¹. Der Rest Y³ ist bevorzugt eine Carboxylgruppe.

Die substituierten Indolverbindungen der allgemeinen Formel II weisen bevorzugt die Formel II' 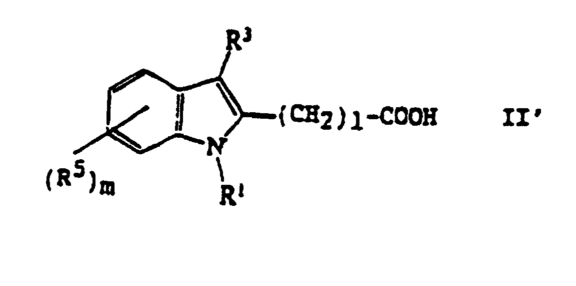 auf. Die bevorzugten Bedeutungen des Restes R³ sind wie vorstehend im Zusammenhang mit Formel I und I' erläutert. Index 1 in Formel II' weist bevorzugt den Wert 0 auf.

Besonders bevorzugt ist der Rest R¹ in Formel II bzw. II' ein Rest Y¹-Ar-Y²-COOH. Der Rest Y¹ ist hier bevorzugt ein C₁-C₄-Alkyl- oder Alkoxyrest und besonders bevorzugt eine Methylen-, Ethylen-, Methoxy- oder Ethoxygruppe. Der Rest Ar ist bevorzugt eine nicht substituierte Phenylengruppe. Der Rest Y² ist bevorzugt ein C₁₋₄-Alkyl-, C₂₋₄-Alkenyl- oder C₁₋₄-Alkoxyrest, wobei Methylen-, Methoxy-, Ethylen- und Ethenylengruppen bevorzugt sind. Bevorzugt steht der Rest Y² in meta- oder para-Stellung zu dem Rest Y¹.

Alle Reste R⁵ sind bevorzugt Wasserstoffatome oder ein Rest R⁵ ist ein Halogenatom, insbesondere 4- oder 5-Chloratom, oder eine Alkoxy-, insbesondere 5-Methoxygruppe.

Beinhalten die Verbindungen der Formel I oder II auch Aminooder Dialkylaminogruppen, so umfaßt die vorliegende Erfindung auch deren Salze, insbesondere deren Hydrochloride.

Die erfindungsgemäßen Verbindungen haben sich als potente Phospholipase A₂-Hemmer erwiesen. Die Verbindungen sind daher brauchbar als Arzneimittel zur Prävention und/oder zur Behandlung von Erkrankungen, die durch Produkte bzw. Folgeprodukte dieses Enzyms verursacht bzw. mitverursacht werden, wie z.B. zur Behandlung des rheumatischen Formenkreises und zur Prävention und Behandlung von allergisch induzierten Erkrankungen. Die erfindungsgemäßen Verbindungen stellen somit u.a. wirksame Analgetika, Antiphlogistika, Antipyretika, Antiallergika und Broncholytika dar und sind zur Thromboseprophylaxe und zur Prophylaxe des anaphylaktischen Schocks sowie zur Behandlung dermatologischer Erkrankungen, wie Psoriasis, Urtikaria, akute und chronische Exantheme allergischer und nichtallergischer Genese, brauchbar.

Die erfindungsgemäßen Verbindungen können entweder als einzelne therapeutische Wirkstoffe oder als Mischungen mit anderen therapeutischen Wirkstoffen verabreicht werden. Sie können alleine verabreicht werden, im allgemeinen werden sie jedoch in Form pharmazeutischer Mittel verabreicht, d.h. als Mischungen der Wirkstoffe mit geeigneten pharmazeutischen Trägern oder Verdünnungsmitteln. Die Verbindungen oder Mittel können oral, parenteral, durch Inhalation oder topisch (einschließlich dermal, transdermal, bukal und sublingual) verabreicht werden.

Die Art des pharmazeutischen Mittels und des pharmazeutischen Trägers bzw. Verdünnungsmittels hängt von der gewünschten Verabreichungsart ab. Orale Mittel können beispielsweise als Tabletten oder Kapseln, auch in retardierter Form, vorliegen und können übliche Exzipienzien enthalten, wie Bindemittel (z.B. Sirup Akazia, Gelatine, Sorbit, Tragant oder Polyvinylpyrrolidon), Füllstoffe (z.B. Lactose, Zucker, Maisstärke, Calciumphosphat, Sorbit oder Glycin), Gleitmittel (z.B. Magnesiumstearat, Talkum, Polyethylenglycol oder Siliciumdioxid), des integrierende Mittel (z.B. Stärke) oder Netzmittel (z.B. Natriumlaurylsulfat). Orale flüssige Präparate können in Form wäßriger oder öliger Suspensionen, Lösungen, Emulsionen, Sirupen, Elixieren oder Sprays usw. vorliegen und können als Trockenpulver zur Rekonstitution mit Wasser oder einem anderen geeigneten Träger vorliegen. Derartige flüssige Präparate können übliche Additive, beispielsweise Suspendiermittel, Geschmacksstoffe, Verdünnungsmittel oder Emulgatoren enthalten. Für die parenterale Verabreichung kann man Lösungen oder Suspensionen mit üblichen pharmazeutischen Trägern einsetzen. Für die Verabreichung durch Inhalation können die Verbindungen in wäßriger oder teilweise wäßriger Lösung vorliegen, die in Form eines Aerosols angewendet werden kann. Mittel für die topische Anwendung können z.B. als pharmazeutisch verträgliche Puder, Lotionen, Salben, Cremes, Gele oder als therapeutische Systeme vorliegen, die therapeutisch wirksame Mengen der erfindungsgemäßen Verbindungen enthalten.

Die erforderliche Dosis ist abhängig von der Form des angewendeten pharmazeutischen Mittels, von der Art der Anwendung, der Schwere der Symptome und dem speziellen Subjekt (Mensch oder Tier), das behandelt wird. Die Behandlung wird üblicherweise mit einer Dosis begonnen, die unterhalb der optimalen Dosis liegt. Danach wird die Dosis erhöht, bis der für die gegebenen Bedingungen optimale Effekt erzielt wird. Im allgemeinen werden die erfindungsgemäßen Verbindungen am besten in Konzentrationen verabreicht, mit welchen sich effektive Wirkungen erzielen lassen, ohne daß schädliche oder nachteilige Wirkungen auftreten. Sie können in einer Einzeldosis oder in mehreren Dosen verabreicht werden .

Die Wirksamkeit der erfindungsgemäßen Verbindungen läßt sich anhand der Hemmung der Phospholipase A₂ bestimmen. Dazu wird in intakten Rinderthrombocyten die Phospholipase A₂ mit Calcium Ionophor A23187 stimuliert und dadurch die Freisetzung von Arachidonsäure aus den Membranphospholipiden ausgelöst. Um die Metabolisierung des Enzymproduktes Arachidonsäure über den Cyclooxygenase-Weg und den 12-Lipoxygenase-Weg zu verhindern, wird dabei der duale Cyclooxygenase/12-Lipoxygenase-Inhibitor 5,8,11,14-Eikosatetrainsäure zugesetzt. Nach Reinigung mittels Festphasenextraktion wird die freigesetzte Arachidonsäure durch reversed *phase*-HPLC mit UV-Detektion bestimmt. Die Hemmung des Enzyms durch eine Testsubstanz ergibt sich aus dem Verhältnis von der in Anwesenheit bzw. in Abwesenheit der Testsubstanz gebildeten Arachidonsäuremengen. Nähere Angaben zum Testsystem erfolgen in dem Beispiel 15.

Die vorliegende Erfindung umfaßt weiterhin Verfahren zur Herstellung der substituierten Pyrrolverbindungen und der substituierten Indolverbindungen.

Die erfindungsgemäßen Verbindungen lassen sich gemäß den folgenden Methoden darstellen.

### Methode 1

Als Ausgangsverbindungen zur Darstellung erfindungsgemäßer Verbindungen eignen sich die Acylpyrrolcarbonsäureester III bzw. die 3-Acylindolcarbonsäureester VI. Diese Ester werden am Indolstickstoff zu den Verbindungen IV bzw. VII alkyliert. Die N-Alkylierung erfolgt beispielsweise wie üblich unter Verwendung der entsprechenden Alkylhalogenide Br-Y¹-Aryl-Y²-COOR²¹ in Gegenwart einer Base, z.B. Alkalimetallalkoholat, wie Kalium-t-butylat, in einem inerten Lösungsmittel, wie DMSO oder dergleichen. Die N-Alkylierung läßt sich auch heterogen unter Verwendung von Phasentranspherkatalysatoren in einem organischen Lösungsmittel, wie Ether, unter Zusatz von gepulvertem Alkalihydroxid, wie Natriumhydroxid, durchführen. Aus IV bzw. VII erhält man durch Esterspaltung die erfindungsgemäßen Carbonsäuren V bzw. VIII. Die Esterspaltung kann hydrolytisch, z.B. mit alkoholischer Kalilauge, oder im Falle der Benzylester auch hydrogenolytisch, z.B. in THF mit Wasserstoff in Gegenwart von Pd/C, erfolgen. Letztere Methode ist vor allem dann angezeigt, wenn neben den Estergruppen weitere hydrolyseempfindliche funktionelle Gruppen in den Verbindungen enthalten sind oder wenn nur eine der beiden Estergruppen gespalten werden soll.

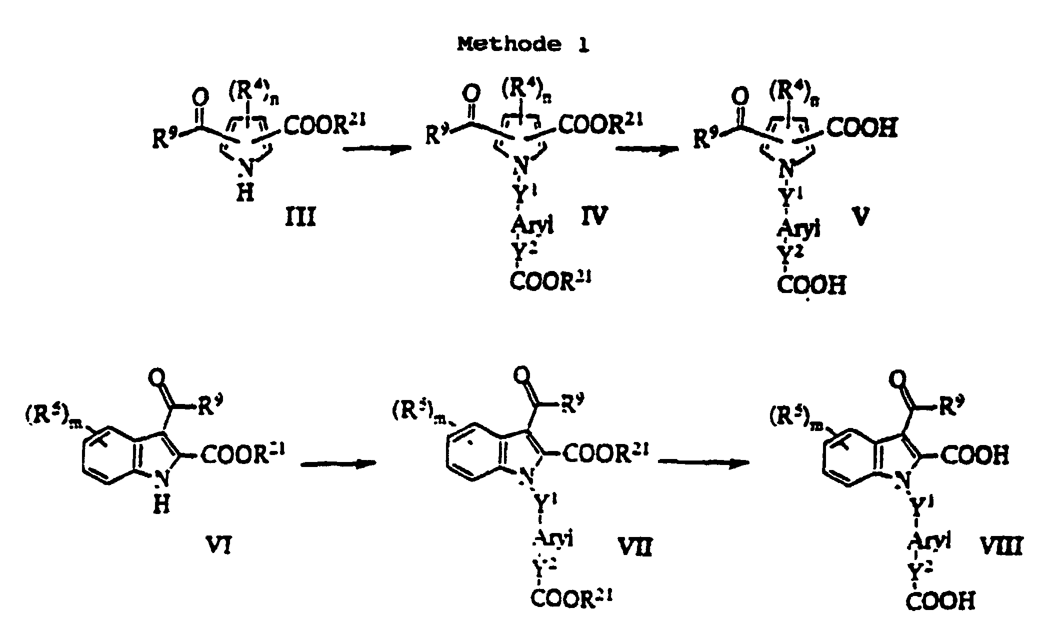

### Methode 2

Erfindungsgemäße Acylpyrrolylcarbonsäuren lassen sich alternativ auch nach Methode 2 darstellen. Dabei werden die Acylpyrrole IX zunächst N-alkyliert gemäß Methode 1. Anschließend wird der Carbonsäurerest eingeführt, z.B. durch Umsetzung mit Chlorameisensäureestern, Diazoessigsäureestern, Acrylsäure bzw. Acrylsäureestern unter Verwendung geeigneter Katalysatoren, wie z.B. Kupfer bei Umsetzung mit Diazoessigsäureestern und AlCl₃ oder BF₃ bei Umsetzung mit Chlorameisensäureestern, Acrylsäure bzw. Acrylsäureestern. Die erhaltenen Verbindungen können schließlich ggf. wie unter Methode 1 beschrieben zu den Carbonsäuren XI bzw. XII hydrolysiert werden.

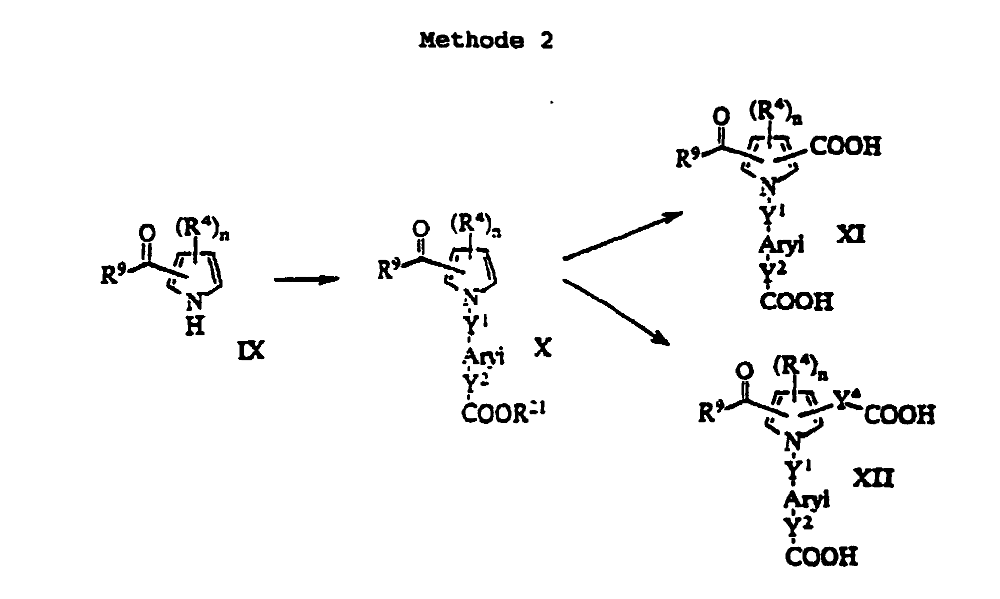

### Methode 3

Erfindungsgemäße Acylpyrrolcarbonsäuren und Acylindolcarbonsäuren können auch mit der in Methode 3 gezeigten Reaktionssequenz ausgehend von Pyrrolcarbonsäureestern XIII bzw. Indolcarbonsäurestern XVII synthetisiert werden. Dabei wird der Pyrrol- bzw. Indolstickstoff zunächst alkyliert gemäß Methode 1. Anschließend wird der Acylrest eingeführt z.B. durch Friedel-Crafts-Acylierung mit Carbonsäurechloriden oder im Falle der Indole auch durch Umsetzung mit Carbonsäuren in Gegenwart von Trifluoressigsäureanhydrid und Polyphosphorsäure, ggf. in einem geeigneten Lösungsmittel, wie z.B. CH₂Cl₂ oder Nitrobenzol (vgl. Murakami et al., Chem. Pharm. Bull. 1985, 33, 4707-4716; Murakami et al., Heterocycles 1980, 14, 1939; Murakami et al., Heterocycles 1984, 22, 241-244; Murakami et al., Chem. Pharm. Bull. 1988, 36, 2023-2035; Tani et al., Chem. Pharm. Bull. 1990, 38, 3261-3267). Die erhaltenen Verbindungen XV und XIX werden ggf. schließlich wie unter Methode 1 beschrieben zu den Carbonsäuren XVI und XX hydrolysiert.

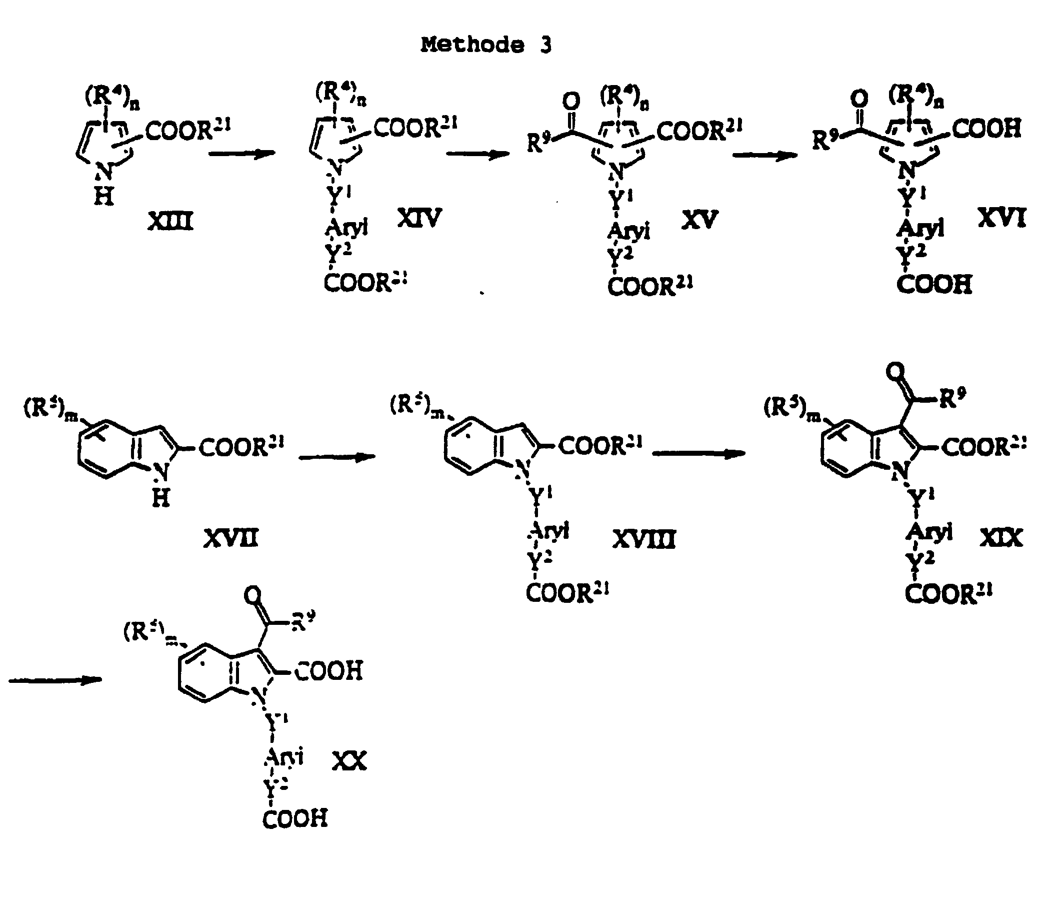

Die bei den Methoden 1 bis 3 in den Zwischen- bzw. Endprodukten auftretenden -COOH und -COOR²¹ Gruppen können unabhängig voneinander mit Hilfe in der Literatur beschriebener Methoden in -CONR¹⁷R¹⁷, -CONHCOR¹⁹, -CONHS(O)₂R¹⁹, -CONHNHS(O)₂R¹⁹ und 1H- bzw. 2H-Tetrazol-5-yl umgewandelt werden.

### Repräsentative Verbindungen

Die Tabellen 1 und 2 zeigen repräsentative Verbindungen der Erfindung.

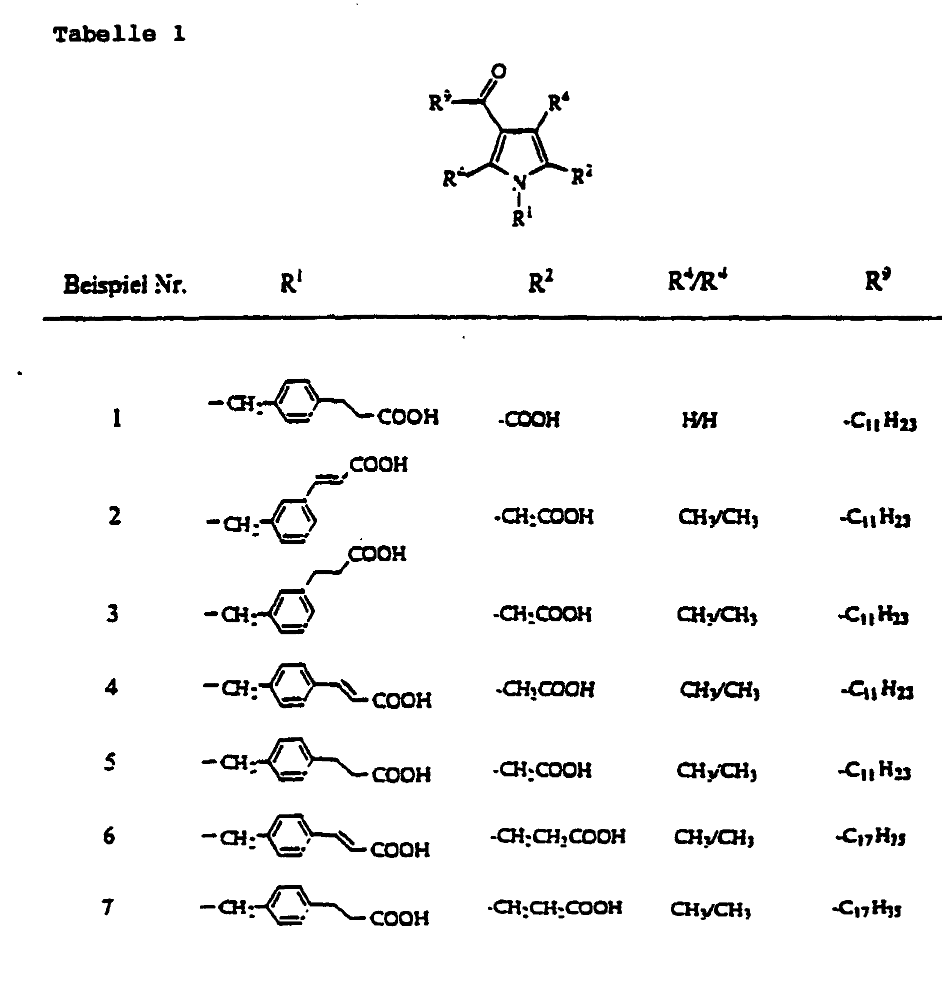

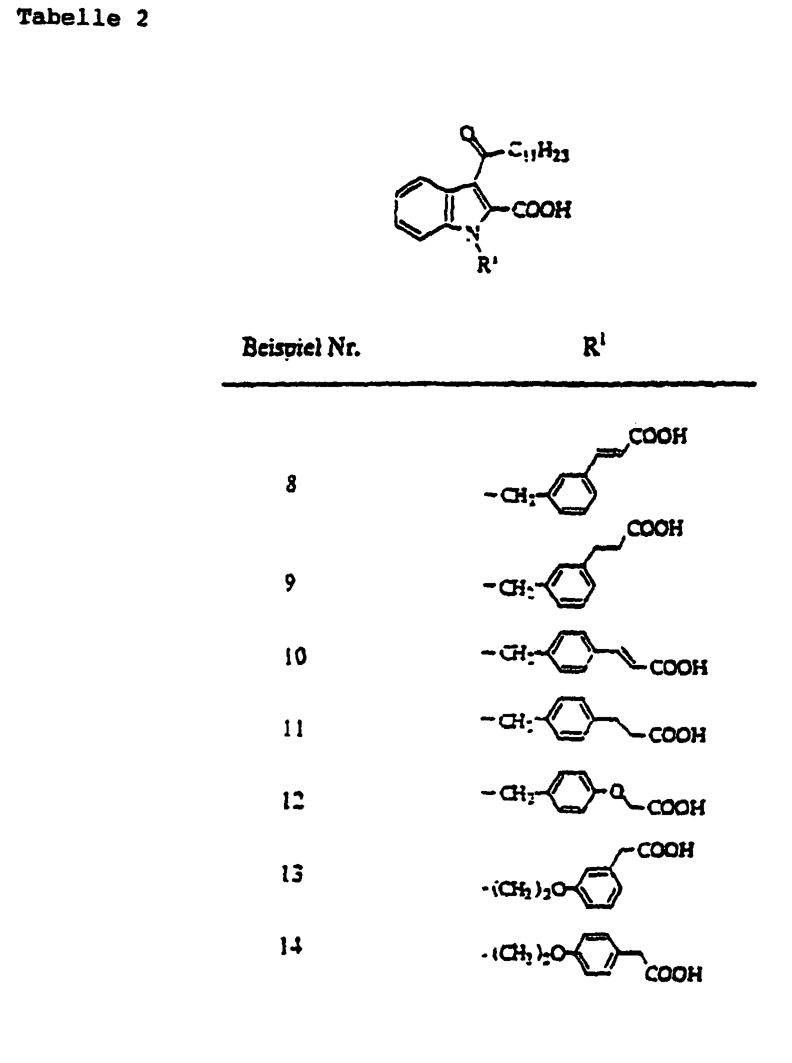

Die nachfolgenden Beispiele erläutern die Erfindung.

Die Ansätze wurden unter Ausschluß von Luftsauerstoff durchgeführt. Zur Säulenchromatographie (SC) wurde Kieselgel 60 (70-230 mesh ASTM) der Fa. Merck, Darmstadt, verwendet; die Substanzen wurden zum Auftragen auf die Säulen in Lösungsmitteln gelöst, deren Elutionsstärke geringer war als die Elutionsstärke des jeweils angegebenen Elutionsmittels (üblicherweise Toluol, CHCl₃ oder CH₂Cl₂ bzw. Mischungen dieser Lösungsmittel mit Petrolether). Alle Temperaturangaben sind unkorrigiert. Bei der Aufnahme der Massenspektren wurde entweder mittels Elektronenstoß (EI) oder chemisch mit CH₄-Gas bzw. CH₅⁺-Ionen (CI) ionisiert. Die NMR-Spektren sind 400 MHz-Spektren, die mit Tetramethylsilan (TMS) als internem Standard vermessen wurden.

### Beispiel 1

### 1-[4-(2-Carboxyethyl)benzyl]-4-dodecanoylpyrrol-2-carbonsäure

### A. 4-Dodecanoylpyrrol-2-carbonsäuremethylester

Die Lösung von 1.25 g (10 mmol) Pyrrol-2-carbonsäuremethylester und 2. 63 g (12 mmol) Dodecansäurechlorid in 30 ml absol. CH₂Cl₂ wird mit 1.46 g (11 mmol) AlCl₃ versetzt und anschließend 24 h gerührt. Nach Zusatz von Wasser wird mit Ether extrahiert. Die organische Phase wird über Na₂SO₄ getrocknet, das Lösungsmittel abdestilliert. Das Produkt wird mittels SC (Kieselgel, 1. Petrolether/Ethylacetat 9+1, 2. CHCl₃, 3. Petrolether/Ethylacetat 8+2) isoliert und aus Petrolether ausgefällt.
Ausbeute: 1.2 g (39 %)
Schmp.: 92-94°C
C₁₈H₂₉NO₃ (307.4)
MS (CI): m/z (rel.Int.) = 308 (100 %), 277 (5 %), 250 (7 %) ¹H-NMR (CDCl₃): δ (ppm) = 0.88 (t, J = 7 Hz, 3H, CH₃), 1.14-1.39 (m, 16H, (CH₂)₈), 1.70 (quint, J = 7 Hz, 2H, CH₂CH₂CO), 2.75 (t, J = 7 Hz, 2H, CH₂CH₂CO), 3.89 (s, 3H, OCH₃), 7.29 (s, 1H, aromat. H), 7.54 (s, 1H, aromat. H), 9.40 (breit, 1H, NH)

### B. (E)-4-Dodecanoyl-1-{4-[2-(ethoxycarbonyl)ethen-1-yl]benzyl}pyrrol-2-carbonsäuremethylester

Die Mischung aus 307 mg (1 mmol) 4-Dodecanoylpyrrol -2-carbonsäuremethylester, 124 mg (1.1 mmol) Kalium-t-butylat und 3 ml absol. DMSO wird 5 min im Ölbad bei 110°C gerührt. Anschließend gibt man 296 mg (1.1 mmol) (*E*)-4-(Brommethyl)zimtsäureethylester (Wang J.-Y, Jun Y.-F. CA 62:1592a) zu und erhitzt den Ansatz weitere 10 min bei gleicher Temperatur. Nach dem Abkühlen wird mit Wasser und NaCl versetzt und mit Ether extrahiert. Die organische Phase wird über Na₂SO₄ getrocknet, das Lösungsmittel abdestilliert und das Produkt mittels SC (Kieselgel, Petrolether/Ethylacetat 9+1) isoliert. Die Produktfraktionen werden eingeengt; das verbleibende Öl kristallisiert nach einiger Zeit.
Ausbeute: 252 mg (51 %)
Schmp.: 79-80°C
C₃₀H₄₁NO₅ (495.7)
MS (EI): m/z (rel.Int.) = 495 (12 %), 355 (100 %), 189 (60 %), 115 (29 %)
¹H-NMR (CDCl₃): δ (ppm) = 0.88 (t, J = 7 Hz, 3H, CH₃), 1.17-1.39 (m, 16H, (CH₂)₈), 1.33 (t, J = 7 Hz, 3H, OCH₂CH₃), 1.69 (quint, J = 7 Hz, 2H, CH₂CH₂CO), 2.73 (t, J = 7 Hz, 2H, CH₂CH₂CO), 3.80 (s, 3H, OCH₃), 4.26 ( q, J = 7 Hz, 2H, OCH₂CH₃), 5.57 (s, 2H, NCH₂), 6.41 (d, J = 16 Hz, 1H, CH=CHCO), 7.14 (d, J = 8 Hz, 2H, aromat. H), 7.38 (d, J = 2 Hz, 1H, aromat. H), 7.48 (d, J = 8 Hz, 2H, aromat. H), 7.48 (d, J = 2 Hz, 1H, aromat. H), 7.64 (d, J = 16 Hz, 1H, CH=CHCO)

### C. 1-[4-(2-Carboxyethyl)benzyl]-4-dodecanoylpyrrol-2-carbonsäure

50 mg (0.1 mmol) (*E*)-4-Dodecanoyl-1-{4-[2-(ethoxycarbonyl)-ethen-1-yl]benzyl}pyrrol-2-carbonsäuremethylester werden in 5 ml THF gelöst. Nach Zusatz einer Spatelspitze Pd/C hydriert man unter kräftigem Rühren 4 h bei Raumtemperatur unter Wasserstoffatmosphäre, die mit Hilfe eines auf den Reaktionskolben aufgesetzten mit Wasserstoff gefüllten Luftballons erzeugt wird. Nach Zusatz von Kieselgur wird filtriert, das Lösungsmittel abdestilliert, der Rückstand mit 12 ml Ethanol und 4 ml 10%-iger wäßriger KOH versetzt und die erhaltene Mischung 1 h unter Rückfluß zum Sieden erhitzt. Nach dem Abkühlen verdünnt man mit Wasser, säuert mit verd. HCl an und extrahiert mit Ether. Die organische Phase wird mit verd. HCl gewaschen, über Na₂SO₄ getrocknet und eingeengt. Das Produkt wird aus Petrolether ausgefällt.
Ausbeute: 26 mg (57 %)
Schmp.: 131-132°C
C₂₇H₃₇NO₅ (455.6)
¹H-NMR (CDCl₃): δ (ppm) = 0.88 (t, J = 7 Hz, 3H, CH₃), 1.13-1.32 (m, 16H, (CH₂)₈), 1.54 (quint, J = 7 Hz, 2H, CH₂CH₂CO), 2.42 (t, J = 7 Hz, 2H, CH₂), 2.68 ( t, J = 7 Hz, 2H, CH₂), 2.91 (t, J = 7 Hz, 2H, CH₂), 5.44 (s, 2H, NCH₂), 6.74 (d, J = 2 Hz, 1H, aromat. H), 6.91 (d, J = 8 Hz, 2H, aromat. H), 6.96 (d, J = 2 Hz, 1H, aromat. H), 7.12 (d, J = 8 Hz, 2H, aromat. H)

### Beispiel 2

### (E)-3-{[2-(Carboxymethyl)-4-dodecanoyl-3,5-dimethylpyrrol-1-yl]methyl}zimtsäure

### A. 4-Dodecanoyl-3,5-dimethylpyrrol-2-carbonsäureethylester

Die Lösung von 1.67 g (10 mmol) 3,5-Dimethylpyrrol-2-carbonsäureethylester und 2.63 g (12 mmol) Dodecansäurechlorid in 30 ml absol. Dichlorethan wird mit 1.46 g (11 mmol) AlCl₃ versetzt und anschließend 24 h gerührt. Nach Zusatz von Wasser und verd. HCl wird zweimal mit CH₂Cl₂ extrahiert. Die organischen Phasen werden mit verd. NaOH gewaschen, über Na₂SO₄ getrocknet und eingeengt. Das Produkt wird aus Isopropanol umkristallisiert.
Ausbeute: 1.45 g (41 %)
Schmp.: 85-87°C
C₂₁H₃₅NO₃ (349.5)
MS (EI): m/z (rel.Int.) = 349 (12 %), 209 (86 %), 194 (100 %), 148 (97 %)
¹H-NMR (CDCl₃): δ (ppm) = 0.88 (t, J = 7 Hz, 3H, CH₃), 1.18-1.42 (m, 16H, (CH₂)₈), 1.37 (t, J = 7 Hz, 3H, OCH₂CH₃), 1.68 (quint, J = 7 Hz, 2H, CH₂CH₂CO), 2.51 (s, 3H, PyrCH₃), 2.59 (s, 3H, PyrCH₃), 2.72 (t, J = 7 Hz, 2H, CH₂CH₂CO), 4.33 (q, J - 7 Hz, 2H, OCH₂CH₃), 8.87 (breit, 1H, NH)

### B. 3-Dodecanoyl-2,4-dimethylpyrrol

Die Mischung aus 1.40 g (4 mmol) 4-Dodecanoyl-3,5-dimethylpyrrol-2-carbonsäureethylester, 30 ml Ethanol und 10 ml 20%-iger wäßriger KOH-Lösung wird zwei Stunden zum Sieden erhitzt. Anschließend versetzt man mit Wasser, säuert mit 10%-iger HCl an und extrahiert zweimal mit CHCl₃. Das Lösungsmittel wird abdestilliert und der Rückstand 20 min im Ölbad bei 160-170°C unter Anlegung eines Wasserstrahlvakuums erhitzt. Das Produkt wird durch SC (Aluminiumoxid neutral, Akt. I, Petrolether/Ethylacetat 9+1) isoliert und aus Petrolether ausgefällt.
Ausbeute: 0.85 g (76 %)
Schmp.: 59-60°C
C₁₈H₃₃NO (279.5)
MS (EI): m/z (rel.Int.) = 277 (12 %), 137 (44 %), 122 (100 %)
¹H-NMR (CDCl₃): δ (ppm) = 0.88 (t, J = 7 Hz, 3H, CH₃), 1.18-1.42 (m, 16H, (CH₂)₈), 1. 68 (quint, J = 7 Hz, 2H, CH₂CH₂CO), 2.28 ( s, 3H, PyrCH₃), 2.50 (s, 3H, PyrCH₃), 2.71 (t, J = 7 Hz, 2H, CH₂CH₂CO), 6.36 (s, 1H, aromat. H), 7.92 (breit, 1H, NH)

### C. (E)-3-[(3-Dodecanoyl-2,4-dimethylpyrrol-1-yl)methyl]zimtsäureethylester

Die Mischung aus 277 mg (1 mmol) 3-Dodecanoyl-2,4-dimethylpyrrol, 296 mg (1.1 mmol) (*E*)-3-(Brommethyl) zimtsäureethylester (Wang J.-Y, Jun Y.-F., CA 62:1592a), 35 mg (0.11 mmol) Tetrabutylammoniumbromid, 200 mg pulverisiertem NaOH, 10 ml Ether, 5 ml CH₂Cl₂ und 2 Tropfen Wasser wird 3 h unter kräftigem Rühren zum schwachen Sieden erhitzt. Anschließend wird der Ansatz filtriert und der Filterrückstand mit CH₂Cl₂ gewaschen. Die Filtrate werden über Na₂SO₄ getrocknet, das Lösungsmittel abdestilliert und das entstandene Produkt mittels SC (Kieselgel, Petrolether/Ethylacetat 9+1) isoliert. Die Produktfraktionen werden eingeengt; das verbleibende Öl kristallisiert nach einiger Zeit.
Ausbeute: 272 mg (58 %)
Schmp.: 58-60°C
C₃₀H₄₃NO₃ (465.7)
MS (EI): m/z (rel.Int.) = 465 (23 %), 310 (100 %), 189 (38 %), 122 (15 %)
¹H-NMR (CDCl₃): δ (ppm) = 0.88 (t, J = 7 Hz, 3H, CH₃), 1.20-1.41 (m, 16H, (CH₂)₈), 1.34 (t, J = 7 Hz, 3H, OCH₂CH₃), 1.69 (quint, J = 7 Hz, 2H, CH₂CH₂CO), 2.28 ( s, 3H, PyrCH₃), 2.41 (s, 3H, PyrCH₃), 2.73 (t, J = 7 Hz, 2H, CH₂CH₂CO), 4.26 (q, J = 7 Hz, 2H, OCH₂CH₃), 4.99 (s, 2H, NCH₂), 6.35 (s, 1H, aromat. H), 6.39 (d, J = 16 Hz, 1H, CH=CHCO), 7.01 (d, J = 8 Hz, 1H, aromat. H), 7.15 (s, 1H, aromat. H), 7.34 (t, J = 8 Hz, 1H, aromat. H), 7.45 (d, J = 8 Hz, 1H, aromat. H), 7.63 (d, J = 16 Hz, 1H, CH=CHCO)

### D. (E)-3-({3-Dodecanoyl-5-[(ethoxycarbonyl)methyl]-2,4-dimethylpyrrol-1-yl}methyl)zimtsäureethylester

Zu einer Lösung von 233 mg (0.5 mmol) (*E*)-3-[(3-Dodecanoyl-2,4-dimethylpyrrol-1-yl)methyl]zimtsäureethylester in 3 ml absol. Toluol werden bei 115-120°C Badtemperatur 0.24 ml Diazoessigsäureethylester in Anteilen von jeweils 0.08 ml unter Rühren während 15 min zugesetzt. Dabei wird nach jedem Diazoessigsäureethylester-Zusatz eine Spatelspitze Kupferpulver zugegeben. Anschließend erhitzt man weitere 5 min. Nach dem Erkalten wird der gesamte Ansatz nach Zusatz von etwas Petrolether auf eine Kieselgel-Säule gegeben; die Elution erfolgt mit Petrolether/Ethylacetat 9+1. Die Produktfraktionen werden eingeengt, wobei eine wachsartige Substanz zurückbleibt.
Ausbeute: 119 mg (43 %)
C₃₄H₄₉NO₅ (551.8)
MS (EI): m/z (rel.Int.) = 551 (47 %), 478 (60 %), 396 (100 %), 338 (33 %), 189 (72 %)
¹H-NMR (CDCl₃): δ (ppm) = 0.88 (t, J = 7 Hz, 3H, CH₃), 1.18 (t, J = 7 Hz, 3H, OCH₂CH₃), 1.18-1.41 (m, 16H, (CH₂)₈), 1.34 (t, J = 7 Hz, 3H, OCH₂CH₃), 1.70 (quint, J = 7 Hz, 2H, CH₂CH₂CO), 2.28 (s, 3H, PyrCH₃), 2.41 ( s, 3H, PyrCH₃), 2.76 (t, J = 7 Hz, 2H, CH₂CH₂CO), 3.45 (s, 2H, PyrCH₂COOC₂H₅), 4.01 (q, J = 7 Hz, 2H, OCH₂CH₃), 4.26 (q, J = 7 Hz, 2H, OCH₂CH₃), 5.16 (s, 2H, NCH₂), 6.38 (d, J = 16 Hz, 1H, CH=CHCO), 6.85 (d, J = 8 Hz, 1H, aromat. H), 7.02 (s, 1H, aromat. H), 7.32 (t, J = 8 Hz, 1H, aromat. H), 7.43 (d, J = 8 Hz, 1H, aromat . H), 7.61 (d, J = 16 Hz, 1H, CH=CHCO)

### E. (E)-3-{[2-(Carboxymethyl)-4-dodecanoyl-3,5-dimethylpyrrol-1-yl]methyl}zimtsäure

Die Mischung aus 55 mg (0.1 mmol) (*E*)-3-({3-Dodecanoyl-5-[(ethoxycarbonyl)methyl]-2,4-dimethylpyrrol-1-yl}methyl)zimtsäureethylester, 12 ml Ethanol und 4 ml 10%-iger wäßriger KOH-Lösung wird 1 h zum Sieden erhitzt. Nach dem Abkühlen verdünnt man mit Wasser, säuert mit verd. HCl an und extrahiert mit Ether. Die organische Phase wird mit verd. HCl gewaschen, über Na₂SO₄ getrocknet und eingeengt. Das Produkt wird aus Methanol/H₂O umkristallisiert.
Ausbeute: 14 mg (28 %)
Schmp.: 133-135°C
C₃₀H₄₁NO₅ (495.7)
¹H-NMR (CDCl₃): δ (ppm) = 0.88 (t, J = 7 Hz, 3H, CH₃), 1.18-1.42 (m, 16H, (CH₂)₈), 1.71 (quint, J = 7 Hz, 2H, CH₂CH₂CO), 2.32 (s, 3H, PyrCH₃), 2.41 (s, 3H, PyrCH₃), 2.78 ( t, J = 7 Hz, 2H, CH₂CH₂CO), 3.59 (s, 2H, PyrCH₂COOH), 5 .11 (s, 2H, NCH₂), 6.24 (d, J = 16 Hz, 1H, CH=CHCO), 6.81 (s, 1H, aromat. H), 7.18 (d, J = 8 Hz, 1H, aromat. H), 7.37 (t, *J* = 8 Hz, 1H, aromat. H), 7.48 (d, J = 8 Hz, 1H, aromat. H), 7.52 (d, J = 16 Hz, 1H, CH=CHCO)

### Beispiel 3

### 3-(3-{[2-(Carboxymethyl)-4-dodecanoyl-3,5-dimethylpyrrol-1-yl]methyl}phenyl)propionsäure

Darstellung gemäß Beispiel 1C unter Verwendung von 55 mg (0.1 mmol) (*E*)-3-({3-Dodecanoyl-5-[(ethoxycarbonyl)methyl]-2,4-dimethylpyrrol-1-yl}methyl)zimtsäureethylester (Beispiel 2D) anstelle von (*E*)-4-Dodecanoyl-1-{4-[2-(ethoxycarbonyl)ethen-1-yl]benzyl}pyrrol-2-carbonsäuremethylester. Das Produkt wird aus Methanol/H₂O umkristallisiert.
Ausbeute: 24 mg (48 %)
Schmp.: 94-96°C
C₃₀H₄₃NO₅ (497.7)
¹H-NMR (CDCl₃): δ (ppm) = 0.88 (t, J = 7 Hz, 3H, CH₃), 1.17-1.45 (m, 16H, (CH₂)₈), 1.69 (quint, J = 7 Hz, 2H, CH₂CH₂CO), 2.27 (s, 3H, PyrCH₃), 2.38 (s, 3H, PyrCH₃), 2.59 (t, J = 7 Hz, 2H, CH₂), 2.75 (t, J = 7 Hz, 2H, CH₂), 2.88 (t, J = 7 Hz, 2H, CH₂), 3.52 (s, 2H, PyrCH₂COOH), 5.08 (s, 2H, NCH₂), 6.69 (s, 1H, aromat. H), 6.77 (d, J = 8 Hz, 1H, aromat. H), 7.07 (d, J = 8 Hz, 1H, aromat. H), 7.22 (t, J = 8 Hz, 1H, aromat. H)

### Beispiel 4

### (E)-4-{[2-(Carboxymethyl)-4-dodecanoyl-3,5-dimethylpyrrol-1-yl]methyl}zimtsäure

### A. (E)-4-[(3-Dodecanoyl-2,4-dimethylpyrrol-1-yl)methyl]zimtsäureethylester

Darstellung gemäß Beispiel 2C mit (*E*)-4-(Brommethyl)zimtsäureethylester anstelle von (*E*)-3-(Hrommethyl)zimtsäureethylester.
Ausbeute: 267 mg (57 %)
Schmp.: 57-59°C
C₃₀H₄₃NO₃ (465.7)
MS (EI): m/z (rel.Int.) = 465 (3 %), 310 (17 %), 189 (20 %), 122 (100 %)
¹H-NMR (CDCl₃): δ (ppm) = 0.88 (t, J = 7 Hz, 3H, CH₃), 1.18-1.39 (m, 16H, (CH₂)₈), 1.34 (t, J = 7 Hz, 3H, OCH₂CH₃), 1.69 (quint, J = 7 Hz, 2H, CH₂CH₂CO), 2.28 (s, 3H, PyrCH₃), 2.40 (s, 3H, PyrCH₃), 2.72 ( t, J = 7 Hz, 2H, CH₂CH₂CO), 4.26 (q, J = 7 Hz, 2H, OCH₂CH₃), 4.99 (s, 2H, NCH₂), 6.34 (s, 1H, aromat. H), 6.41 (d, J = 16 Hz, 1H, CH=CHCO), 7.01 (d, J = 8 Hz, 2H, aromat. H), 7.47 (d, J = 8 Hz, 2H, aromat. H), 7.64 (d, J = 16 Hz, 1H, CH=CHCO)

### B. (E)-4-({3-Dodecanoyl-5-[(ethoxycarbonyl)methyl]-2,4-dimethylpyrrol-1-yl}methyl)zimtsäureethylester

Darstellung gemäß Beispiel 2D mit (*E*)-4-[(3-Dodecanoyl-2,4-dimethylpyrrol-1-yl)methyl]zimtsäureethylester anstelle von (*E*)-3-[(3-Dodecanoyl-2,4-dimethylpyrrol-1-yl)methyl]zimtsäureethylester.
Ausbeute: 146 mg (53 %)
Schmp. : 69-71°C
C₃₄H₄₉NO₅ (551.8)
MS (EI): m/z (rel.Int.) = 551 (48 %), 478 (48 %), 396 (84 %), 189 (100 %), 115 (51 %)
¹H-NMR (CDCl₃): δ (ppm) = 0.88 (t, J = 7 Hz, 3H, CH₃), 1.18 (t, J = 7 Hz, 3H, OCH₂CH₃), 1.18-1.39 (m, 16H, (CH₂)₈), 1.34 (t, J = 7 Hz, 3H, OCH₂CH₃), 1.69 (quint, J = 7 Hz, 2H, CH₂CH₂CO), 2.27 (s, 3H, PyrCH₃), 2.40 (s, 3H, PyrCH₃), 2.75 (t, J = 7 Hz, 2H, CH₂CH₂CO), 3.45 (s, 2H, PyrCH₂COOC₂H₅), 4.01 (q, J = 7 Hz, 2H, OCH₂CH₃), 4.26 (q, J = 7 Hz, 2H, OCH₂CH₃), 5.16 (s, 2H, NCH₂), 6.40 (d, J = 16 Hz, 1H, CH=CHCO), 6.88 (d, J = 8 Hz, 2H, aromat. H), 7.45 (d, J = 8 Hz, 2H, aromat. H), 7.63 (d, J = 16 Hz, 1H, CH=CHCO)

### C. (E)-4-{[2-(Carboxymethyl)-4-dodecanoyl-3,5-dimethylpyrrol-1-yl]methyl}zimtsäure

Darstellung gemäß Beispiel 2E mit (*E*)-4-({3-Dodecanoyl-5-[(ethoxycarbonyl)methyl]-2,4-dimethylpyrrol-1-yl}methyl)zimtsäureethylester anstelle von (*E*)-3-({3-Dodecanoyl-5-[(ethoxycarbonyl)methyl]-2,4-dimethylpyrrol-1-yl}methyl)zimtsäureethylester. Das Produkt wird aus Methanol umkristallisiert.
Ausbeute: 12 mg (53 %)
Schmp.: 205-207°C
C₃₀H₄₁NO₅ (495.7)
¹H-NMR ([D₆]-DMSO): δ (ppm) = 0.85 (t, J = 7 Hz, 3H, CH₃), 1.14-1.32 (m, 16H, (CH₂)₈), 1.56 (quint, J = 7 Hz, 2H, CH₂CH₂CO), 2.15 (s, 3H, PyrCH₃), 2.29 (s, 3H, PyrCH₃), 2.67 (t, J = 7 Hz, 2H, CH₂CH₂CO), 3.44 (s, 2H, PyrCH₂COOH), 5.18 (s, 2H, NCH₂), 6.46 (d, J = 16 Hz, 1H, CH=CHCO), 6.92 (d, J = 8 Hz, 2H, aromat. H), 7.54 (d, J = 16 Hz, 1H, CH=CHCO), 7.60 ( d, J = 8 Hz, 2H, aromat. H)

### Beispiel 5

### 3-(4-{[2-(Carboxymethyl)-4-dodecanoyl-3,5-dimethylpyrrol-1-yl]methyl}phenyl)propionsäure

Darstellung gemäß Beispiel 1C unter Verwendung von 55 mg (0.1 mmol) (*E*)-4-({3-Dodecanoyl-5-[(ethoxycarbonyl)methyl]-2,4-dimethylpyrrol-1-yl}methyl)zimtsäureethylester (Beispiel 4B) anstelle von (*E*)-4-Dodecanoyl-1-{4-[2-(ethoxycarbonyl)ethen-1-yl]benzyl}pyrrol-2-carbonsäuremethylester. Das Produkt wird aus Ether/Petrolether ausgefällt.
Ausbeute: 20 mg (48 %)
Schmp.: 127-129°C
C₃₀H₄₃NO₅ (497.7)
¹H-NMR (CDCl₃): δ (ppm) = 0.88 (t, J = 7 Hz, 3H, CH₃), 1.18-1.43 (m, 16H, (CH₂)₈), 1.70 (quint, J = 7 Hz, 2H, CH₂CH₂CO), 2.25 (s, 3H, PyrCH₃), 2.48 (s, 3H, PyrCH₃), 2.60 (t, J = 8 Hz, 2H, CH₂), 2.76 (t, J = 7 Hz, 2H, CH₂), 2.88 (t, J = 8 Hz, 2H, CH₂), 3.50 (s, 2H, PyrCH₂COOH), 5.07 (s, 2H, NCH₂), 6.82 (d, J = 8 Hz, 2H, aromat. H), 7.09 (d, J = 8 Hz, 2H, aromat. H)

### Beispiel 6

### (E)-4-{[2-(2-Carboxyethyl)-3,5-dimethyl-4-octadecanoylpyrrol-1-yl]methyl}zimtsäure

### A. (E)-4-[(2,4-Dimethyl-3-octadecanoylpyrrol-1-yl)methyl]zimtsäureethylester

Darstellung gemäß Beispiel 2C mit 362 mg (1 mmol) 2,4-Dimethyl-3-octadecanoylpyrrol (Lehr M., WO95/13266) anstelle von 3-Dodecanoyl-2,4-dimethylpyrrol und von (*E*)-4-(Brommethyl)zimtsäureethylester anstelle von (*E*)-3-(Brommethyl) zimtsäureethylester.
Ausbeute: 300 mg (55 %)
Schmp.: 53-55°C
C₃₆H₅₅NO₃ (549.8)
MS (EI): m/z (rel.Int.) = 549 (7 %), 325 (31 %), 310 (28 %), 189 (18 %), 122 (100 %)
¹H-NMR (CDCl₃): δ (ppm) = 0.88 (t, J = 7 Hz, 3H, CH₃), 1.16-1.39 (m, 28H, (CH₂)₁₄), 1.34 (t, J = 7 Hz, 3H, OCH₂CH₃), 1.68 (quint, J = 7 Hz, 2H, CH₂CH₂CO), 2.28 (s, 3H, PyrCH₃), 2.40 (s, 3H, PyrCH₃), 2.72 (t, J = 7 Hz, 2H, CH₂CH₂CO), 4.26 (q, J = 7 Hz, 2H, OCH₂CH₃), 4.99 ( s, 2H, NCH₂), 6.35 ( s, 1H, aromat. H), 6.41 (d, J = 16 Hz, 1H, CH=CHCO), 7.01 (d, J = 8 Hz, 2H, aromat. H), 7.47 (d, J = 8 Hz, 2H, aromat. H), 7.64 (d, J = 16 Hz, 1H, CH=CHCO)

### B. (E)-4-({2-[2-(Methoxycarbonyl)ethyl]-3,5-dimethyl-4-octadecanoylpyrrol-1-yl}methyl)zimtsäureethylester

Die Lösung von 275 mg (0.5 mmol) (*E*)-4-[(2,4-Dimethyl-3-octadecanoylpyrrol-1-yl)methyl]zimtsäureethylester und 0.25 ml Acrylsäuremethylester in 5 ml absol. Nitrobenzol wird mit 0.1 ml BF₃-Ethylether-Komplex versetzt. Man rührt 24 h bei Raumtemperatur. Nach Zusatz von gesättigter NaCl-Lösung wird mit Ether extrahiert. Das Lösungsmittel wird nach dem Trocknen über Na₂SO₄ abdestilliert und der verbleibende Rückstand mittels SC (Kieselgel, 1. Petrolether/Ethylacetat 9+1, 2. Petrolether/Ethylacetat 8+2) gereinigt. Die Produktfraktionen werden eingeengt; das zurückbleibende Öl kristallisiert nach einiger Zeit.
Ausbeute: 250 mg (79 %)
Schmp.: 72-74°C
C₄₀H₆₁NO₅ (635.9)
MS (CI): m/z (rel.Int.) = 636 (4 %), 448 (100 %), 252 (84 %) ¹H-NMR (CDCl₃): δ (ppm) = 0.88 (t, J = 7 Hz, 3H, CH₃), 1.13-1.39 (m, 28H, (CH₂)₁₄), 1.34 (t, J = 7 Hz, 3H, OCH₂CH₃), 1. 69 (quint, J = 7 Hz, 2H, CH₂CH₂CO), 2.24 (s, 3H, PyrCH₃), 2.32 (t, J = 7 Hz, 2H, CH₂), 2.37 (s, 3H, PyrCH₃), 2.73 (t, J = 7 Hz, 2H, CH₂), 2.80 ( t, J = 7 Hz, 2H, CH₂), 3.63 (s, 3H, OCH₃), 4.26 (q, J = 7 Hz, 2H, OCH₂CH₃), 5.11 (s, 2H, NCH₂), 6.40 (d, J = 16 Hz, 1H, CH=CHCO), 6.87 (d, J = 8 Hz, 2H, aromat. H), 7.46 (d, J = 8 Hz, 2H, aromat. H), 7.64 (d, J = 16 Hz, 1H, CH=CHCO)

### C. (E)-4-{[2-(2-Carboxyethyl)-3,5-dimethyl-4-octadecanoylpyrrol-1-yl]methyl]}zimtsäure

Darstellung gemäß Beispiel 2E mit 64 mg (0.1 mmol) (*E*)-4-({2-[2-(Methoxycarbonyl)ethyl]-3,5-dimethyl-4-octadecanoylpyrrol-1-yl}methyl)zimtsäureethylester anstelle von (*E*)-3-({3-Dodecanoyl-5-[(ethoxycarbonyl)methyl]-2,4-dimethylpyrrol-1-yl}methyl)zimtsäureethylester. Das Produkt wird aus Ether/Petrolether ausgefällt.
Ausbeute: 27 mg (45 %)
Schmp.: 166-168°C
C₃₇H₅₅NO₅ (593.8)
¹H-NMR (CDCl₃): δ (ppm) = 0.88 (t, J = 7 Hz, 3H, CH₃), 1.17-1.42 (m, 28H, (CH₂)₁₄), 1.69 (quint, J = 7 Hz, 2H, CH₂CH₂CO), 2.26 (s, 3H, PyrCH₃), 2.31 (s, 3H, PyrCH₃), 2.47 (t, J = 7 Hz, 2H, CH₂), 2.74 (t, J = 7 Hz, 2H, CH₂), 2.90 (t, J = 7 Hz, 2H, CH₂), 5 .16 (s, 2H, NCH₂), 6 .25 (d, J = 16 Hz, 1H, CH=CHCO), 6.85 (d, J = 8 Hz, 2H, aromat . H), 7 .44 (d, J = 8 Hz, 2H, aromat. H), 7.57 (d, J = 16 Hz, 1H, CH=CHCO)

### Beispiel 7

### 3-(4-{[2-(2-Carboxyethyl)-3,5-dimethyl-4-octadecanoylpyrrol-1-yl]methyl}phenyl)propionsäure

Darstellung gemäß Beispiel 1C unter Verwendung von 64 mg (0.1 mmol) (*E*)-4-({2-[2-(Methoxycarbonyl)ethyl]-3,5-dimethyl-4-octadecanoylpyrrol-lyl}methyl)ztmtsäureethylester (Beispiel 6B) anstelle von (*E*)-4-Dodecanoyl-1-{4-[2-(ethoxycarbonyl)-ethen-1-yl)benzyl}pyrrol-2-carbonsäuremethylester. Das Produkt wird aus Ether/Petrolether ausgefällt.
Ausbeute: 13 mg (22 %)
Schmp.: 152-154°C
C₃₇H₅₇NO₅ (595.9)
¹H-NMR (CDCl₃): δ (ppm) = 0.88 (t, J = 7 Hz, 3H, CH₃), 1.18-1.42 (m, 28H, (CH₂)₁₄), 1. 69 ( quint, J = 7 Hz, 2H, CH₂CH₂CO), 2.19-2.26 (m, 5H, CH₂ und PyrCH₃), 2.39 (s, 3H, PyrCH₃), 2.68 (t, J = 7 Hz, 2H, CH₂), 2.73 (t, J = 7 Hz, 2H, CH₂), 2.80 ( t, J = 7 Hz, 2H, CH₂), 2 .92 ( t, J = 7 Hz, 2H, CH₂), 5.02 (s, 2H, NCH₂), 6.78 (d, J = 8 Hz, 2H, aromat. H), 7.14 (d, J = 8 Hz, 2H, aromat. H)

### Beispiel 8

### (E)-1-[3-(2-Carboxyethen-1-yl)benzyl]-3-dodecanoylindol-2-carbonsäure

### A. 3-Dodecanoylindol-2-carbonsäureethylester

Die Mischung aus 3.8 g (20 mmol) Indol-2-carbonsäureethylester, 6.0 g (30 mmol) Octadecansäure, 1.0 g Polyphosphorsäure, 20 ml absol. CH₂Cl₂ und 4.4 ml Trifluoressigsäureanhydrid wird 4 h bei Raumtemperatur gerührt. Anschließend versetzt man mit 1 M NaOH und extrahiert mit Ether. Die Etherphase wird über Na₂SO₄ getrocknet und das Lösungsmittel abdestilliert. Nach Zusatz von Petrolether fällt das Produkt aus.
Ausbeute: 4.3 g (58 %)
Schmp.: 75-76°C
C₂₃H₃₃NO₃ (371.5)
MS (EI): m/z (rel.Int.) = 371 (6 %), 298 (60 %), 216 (100 %), 188 (42 %), 170 (53 %)
¹H-NMR (CDCl₃): δ (ppm) = 0.88 (t, J = 7 Hz, 3H, CH₃), 1.13-1.39 (m, 16H, (CH₂)₈), 1.43 (t, J = 7 Hz, 3H, OCH₂CH₃), 1.74 (quint, J = 7 Hz, 2H, CH₂CH₂CO), 3.06 (t, J = 7 Hz, 2H, CH₂CH₂CO), 4 .45 ( q, J = 7 Hz, 2H, OCH₂CH₃), 7.24 ( t, J = 8 Hz, 1H, aromat. H), 7.36 (d, J = 8 Hz, 1H, aromat. H), 7.41 (t, J = 8 Hz, 1H, aromat. H), 7.92 (d, J = 8 Hz, 1H, aromat. H), 9.02 (s, 1H, NH)

### B. (E)-3-Dodecanoyl-1-{3-(2-(ethoxycarbonyl)ethen-1-yl]benzyl}indol-2-carbonsäureethylester

Darstellung gemäß Beispiel 1B unter Verwendung von 372 mg (1 mmol) 3-Dodecanoylindol-2-carbonsäureethylester anstelle von 4-Dodecanoylpyrrol-2-carbonsäuremethylester und von (*E*)-3-(Brommethyl)zimtsäureethylester anstelle von (*E*)-4-(Brommethyl)zimtsäureethylester. Das Produkt fällt als wachsartige Substanz an.
Ausbeute: 393 mg (70 %)
C₃₅H₄₅NO₅ (559.7)
MS (EI): m/z (rel.Int.) = .559 (11 %), 486 (39 %), 419 (100 %), 189 (86 %), 115 (38 %)
¹H-NMR (CDCl₃): δ (ppm) = 0.88 (t, J - 7 Hz, 3H, CH₃), 1.18-1.48 (m, 22H, (CH₂)₈ und OCH₂CH₃ und OCH₂CH₃), 1.77 (quint, J = 7 Hz, 2H, CH₂CH₂CO), 2.94 (t, J = 7 Hz, 2H, CH₂CH₂CO), 4.25 (q, J = 7 Hz, 2H, OCH₂CH₃), 4.36 ( q, J = 7 Hz, 2H, OCH₂CH₃), 5.57 (s, 2H, NCH₂), 6.37 ( d, J = 16 Hz, 1H, CH=CHCO), 7 .09 (d, J = 8 Hz, 1H, aromat. H), 7.21-7.34 (m, 5H, aromat. H), 7.43 (d, J = 8 Hz, 1H, aromat. H), 7.59 (d, J = 16 Hz, 1H, CH=CHCO), 7.96 (d, J = 8 Hz, 1H, aromat. H)

### C. (E)-1-[3-(2-Carboxyethen-1-yl)benzyl]-3-dodecanoylindol-2-carbonsäure

Darstellung gemäß Beispiel 2E mit 5 6 mg (0.1 mmol) (*E*)-3-Dadecanoyl-1-{3-[2-(ethoxycarbonyl)ethen-1-yl]benzyl}indol-2-carbonsäureethylester anstelle von (*E*)-3-({3-Dodecanoyl-5-[(ethoxycarbonyl)methyl]-2,4-dimethylpyrrol-1-yl}methyl)zimtsäureethylester. Das Produkt wird aus Ether ausgefällt.
Ausbeute: 21 mg (42 %)
Schmp.: 186-187°C
C₃₁H₃₇NO₅ (503.6)
¹H-NMR ([D₆]-DMSO): δ (ppm) = 0.85 (t, J = 7 Hz, 3H, CH₃), 1.12-1.37 (m, 16H, (CH₂)₈), 1.63 (quint, J = 7 Hz, 2H, CH₂CH₂CO), 2.91 (t, J = 7 Hz, 2H, CH₂CH₂CO), 5.63 (s, 2H, NCH₂), 6.45 ( d, J = 16 Hz, 1H, CH=CHCO), 7 .10 (d, J = 8 Hz, 1H, aromat. H), 7.22-7.34 (m, 3H, aromat. H), 7.51 (d, J = 16 Hz, 1H, CH=CHCO), 7.53-7.60 (m, 3H, aromat. H), 7.96 (d, J = 8 Hz, 1H, aromat . H)

### Beispiel 9

### 1-[3-(2-Carboxyethyl)benzyl]-3-dodecanoylindol-2-carbonsäure

Darstellung gemäß Beispiel 1C unter Verwendung von 56 mg (0.1 mmol) (*E*)-3-Dodecanoyl-1-{3-[2-(ethoxycarbonyl)ethen-1-yl]benzyl}indol-2-carbonsäureethylester (Beispiel 8B) anstelle von (*E*)-4-Dodecanoyl-1-{4-[2-(ethoxycarbonyl)ethen-1-yl]benzyl}pyrrol-2-carbonsäuremethylester. Das bei der Hydrierung entstehende zwischenprodukt wird mit SC (Kieselgel, Petrolether/Essigester 9+1) gereinigt. Das Produkt wird aus Ether ausgefällt.
Ausbeute: 11 mg (22 %)
Schmp.: 131-132°C
C₃₁H₃₉NO₅ (505.7)
¹H-NMR ([D₆]-DMSO): δ (ppm) = 0.85 (t, J = 7 Hz, 3H, CH₃), 1.14-1.37 (m, 16H, (CH₂)₈), 1.63 (quint, J = 7 Hz, 2H, CH₂CH₂CO), 2.48 (t, J = 8 Hz, 2H, CH₂), 2.76 (t, J = 8 Hz, 2H, CH₂), 2.90 ( t, J = 7 Hz, 2H, CH₂CH₂CO), 5.57 (s, 2H, NCH₂), 6.86 (d, J = 8 Hz, 1H, aromat. H), 7.10 (d, J = 8 Hz, 1H, aromat. H), 7.15-7.31 (m, 4H, aromat. H), 7.55 (d, J = 8 Hz, 1H, aromat . H), 7.96 (d, J = 8 Hz, 1H, aromat . H)

### Beispiel 10

### (E)-1-[4-(2-Carboxyethen-1-yl)benzyl]-3-dodecanoylindol-2-carbonsäure

### A. (E)-1-{4-[2-(Ethoxycarbonyl)ethen-1-yl]benzyl}indol-2-carbonsäureethylester

Die Mischung aus 378 mg (2 mmol) Indol-2-carbonsäureethylester, 269 mg (2.4 mmol) Kalium-t-butylat und 5 ml absol. DMSO wird 5 min im Ölbad bei 110°C gerührt. Anschließend gibt man 538 mg (2 mmol) (*E*)-4-(Brommethyl)zimtsäureethylester zu und erhitzt den Ansatz weitere 10 min bei gleicher Temperatur. Nach dem Abkühlen wird mit Wasser und NaCl versetzt und mehrfach mit CHCl₃ extrahiert. Die organischen Phasen werden über Na₂SO₄ getrocknet, das Lösungsmittel abdestilliert und das Produkt mittels SC (Kieselgel, Petrolether/Ethylacetat 9+1) isoliert. Die Produktfraktionen werden eingeengt; das verbleibende Öl kristallisiert nach einiger Zeit.
Ausbeute: 479 mg ( 63 %)
Schmp.: 89-91°C
C₂₃H₂₃NO₄ (377.4)
MS (EI): m/z (rel.Int.) = 377 (18 %), 189 (100 %), 115 (67 %)
¹H-NMR (CDCl₃): δ (ppm) = 1.32 (t, J = 7 Hz, 3H, OCH₂CH₃), 1.36 (t, J = 7 Hz, 3H, OCH₂CH₃), 4.24 (q, J = 7 Hz, 2H, OCH₂CH₃), 4.32 (q, J = 7 Hz, 2H, OCH₂CH₃), 5.85 (s, 2H, NCH₂), 6.35 (d, J = 16 Hz, 1H, CH=CHCO), 7.05 (d, J = 8 Hz, 2H, aromat. H), 7.16-7.20 (m, 1H, aromat. H), 7.29-7.34 (m, 2H, aromat. H), 7.40 (d, J = 8 Hz, 2H, aromat. H), 7.41 (s, 1H, aromat. H), 7.61 (d, J = 16 Hz, 1H, CH=CHCO), 7.72 (d, J = 8 Hz, 1H, aromat. H)

### B. (E)-3-Dodecanoyl-1-{4-[2-(ethoxycarbonyl)ethen-1-yl]benzyl}indol-2-carbonsäureethylester

Die Mischung aus 377 mg (1 mmol) 1-{4-[2-(Ethoxycarbonyl)ethen-1-yl]benzyl}indol-2-carbonsäureethylester, 300 mg (1.5 mmol) Dodecansäure, 67 mg Polyphosphorsäure, 5 ml absol. CH₂Cl₂ und 0.33 ml Trifluoressigsäureanhydrid wird 4 h bei Raumtemperatur gerührt. Anschließend versetzt man mit 1 M NaOH und extrahiert mit Ether. Die organische Phase wird über Na₂SO₄ getrocknet, das Lösungsmittel abdestilliert und das Produkt mittels SC (Kieselgel, Petrolether/Ethylacetat 9+1) isoliert. Die Produktfraktionen werden eingeengt; das verbleibende Öl kristallisiert nach einiger Zeit.
Ausbeute: 219 mg (39 %)
Schmp.: 72-74°C
C₃₅H₄₅NO₅ (559.7)
MS (EI): m/z (rel.Int.) = 559 (12 %), 486 (35 %), 419 (75 %), 189 (100 %), 115 (42 %)
¹H-NMR (CDCl₃): δ (ppm) = 0.88 (t, J = 7 Hz, 3H, CH₃), 1.17-1.45 (m, 16H, (CH₂)₈), 1.28 (t, J = 7 Hz, 3H, OCH₂CH₃), 1.32 (t, J = 7 Hz, 3H, OCH₂CH₃), 1.76 (quint, J = 7 Hz, 2H, CH₂CH₂CO), 2.93 (t, J = 7 Hz, 2H, CH₂CH₂CO), 4.25 (q, J = 7 Hz, 2H, OCH₂CH₃), 4.35 (q, J = 7 Hz, 2H, OCH₂CH₃), 5.59 (s, 2H, NCH₂), 6.38 (d, J = 16 Hz, 1H, CH=CHCO), 7.10 (d, J = 8 Hz, 2H, aromat. H), 7.26-7.35 (m, 3H, aromat. H), 7.44 (d, J = 8 Hz, 2H, aromat. H), 7.62 (d, J = 16 Hz, 1H, CH=CHCO), 7.95 (d, J = 8 Hz, 1H, aromat. H)

### C. (E)-1-[4-(2-Carboxyethen-1-yl]benzyl]-3-dodecanoylindol-2-carbonsäure

Darstellung gemäß Beispiel 2E mit 56 mg (0.1 mmol) (*E*)-3-Dodecanoyl-1-{4-[2-(ethoxycarbonyl)ethen-1-yl]benzyl}indol-2-carbonsäureethylester anstelle von (*E*)-3-({3-Dodecanoyl-5-[(ethoxycarbonyl)methyl]-2,4-dimethylpyrrol-1-yl}methyl)zimtsäureethylester. Das Produkt wird aus Ether ausgefällt.
Ausbeute: 15 mg (30 %)
Schmp.: 224-230°C
C₃₁H₃₇NO₅ (503.6)
¹H-NMR ([D₆]-DMSO): δ (ppm) = 0.85 (t, J = 7 Hz, 3H, CH₃), 1.12-1.36 (m, 16H, (CH₂)₈), 1.63 (quint, J = 7 Hz, 2H, CH₂CH₂CO), 2.91 (t, J = 7 Hz, 2H, CH₂CH₂CO), 5.65 (s, 2H, NCH₂), 6.46 (d, J = 16 Hz, 1H, CH=CHCO), 7.15 (d, J = 8 Hz, 2H, aromat. H), 7.24 (t, J = 8 Hz, 1H, aromat. H), 7.29 (t, J = 8 Hz, 1H, aromat. H), 7.50-7.57 (m, 2H, CH=CHCO und aromat. H), 7.60 (d, J = 8 Hz, 2H, aromat. H), 7.96 (d, J = 8 Hz, 1H, aromat. H)

### Beispiel 11

### 1-[4-(2-Carboxyethyl)benzyl]-3-dodecanoylindol-2-carbonsäure

Darstellung gemäß Beispiel 1C unter Verwendung von 56 mg (0.1 mmol) (*E*)-3-Dodecanoyl-1-{4-[2-(ethoxycarbonyl)ethen-1-yl]benzyl}indol-2-carbonsäureethylester (Beispiel 10B) anstelle von (*E*)-4-Dodecanoyl-1-{4-[2-(ethoxycarbonyl)ethen-1-yl]benzyl}pyrrol-2-carbonsäuremethylester. Das bei der Hydrierung entstehende Zwischenprodukt wird mit SC (Kieselgel, Petrolether/Essigester 1. 9+1, 2. 8+2) gereinigt. Das Produkt wird aus Petrolether ausgefällt.
Ausbeute: 17 mg (34 %)
Schmp.: 168-169°C
C₃₁H₃₉NO₅ (505.7)
¹H-NMR (CDCl₃): δ (ppm) = 0.88 (t, J = 7 Hz, 3H, CH₃), 1.18-1.42 (m, 14H, (CH₂)₇), 1.48 (quint, J = 7 Hz, 2H, CH₂CH₂CH₂CO), 1.88 (quint, J = 7 Hz, 2H, CH₂CH₂CO), 2.62 (t, J = 8 Hz, 2H, CH₂), 2.89 (t, J = 8 Hz, 2H, CH₂), 3.31 ( t, J = 7 Hz, 2H, CH₂), 6.08 (s, 2H, NCH₂), 7.03 ( d, J = 8 Hz, 2H, aromat. H), 7.10 (d, J = 8 Hz, 2H, aromat. H), 7.41-7.46 (m, 2H, aromat. H), 7.54-7.58 (m, 1H, aromat. H), 8.02-8.05 (m, 2H, aromat. H)

### Beispiel 12

### 1-[4-(Carboxymethoxy)benzyl]-3-dodecanoylindol-2-carbonsäure

Die Mischung aus 223 mg (0.6 mmol) 3-Dodecanoylindol-2-carbonsäureethylester, 81 mg (0.72 mmol) Kalium-t-butylat und 2 ml absol. DMSO wird 5 min im Ölbad bei 110°C gerührt. Anschließend gibt man 197 mg (0.72 mmol) 2-[4-(Brommethyl)phenoxy]essigsäureethylester zu und erhitzt den Ansatz weitere 10 min bei gleicher Temperatur. Nach dem Abkühlen wird mit Wasser und NaCl versetzt und mit Ether extrahiert. Die organische Phase wird über Na₂SO₄ getrocknet, das Lösungsmittel abdestilliert und der Rückstand an Kieselgel mit Petrolether/Ethylacetat (9+1) chromatographiert. Der erhaltene 3-Dodecanoyl-1-{4-[(ethoxycarbonyl)methoxy]benzyl}indol-2-carbonsäureethylester wird analog Beispiel 2E verseift. Das Produkt wird aus Ether ausgefällt.
Ausbeute: 60 mg (20 %)
Schmp.: 191-193°C
C₃₀H₃₇NO₆ (507.6)
¹H-NMR (CDCl₃): δ (ppm) = 0.88 (t, J = 7 Hz, 3H, CH₃), 1.18-1.42 (m, 14H, (CH₂)₇), 1.48 (quint, J = 7 Hz, 2H, CH₂CH₂CH₂CO), 1.88 (quint, J = 7 Hz, 2H, CH₂CH₂CO), 3.31 (t, J = 7 Hz, 2H, CH₂CH₂CO), 4.61 (s, 2H, OCH₂CO), 6.06 (s, 2H, NCH₂), 6.82 (d, J = 9 Hz, 2H, aromat. H), 7.10 (d, J = 9 Hz, 2H, aromat. H), 7.43-7.47 (m, 2H, aromat. H), 7.56-7.59 (m, 1H, aromat. H), 8.02-8.05 (m, 2H, aromat. H)

### Beispiel 13

### 1-{2-[3-(Carboxymethyl)phenoxy]ethyl}-3-dodecanoylindol-2-carbonsäure

Darstellung gemäß Beispiel 12 unter Verwendung von 207 mg (0.72 mmol) 2-[3-(2-Bromethoxy)phenyl]essigsäureethylester anstelle von 2-[4-(Brommethyl)phenoxy]essigsäureethylester. Das Produkt wird aus Ether/Petrolether ausgefällt.
Ausbeute: 84 mg (27 %)
Schmp.: 114-116°C
C₃₁H₃₉NO₆ (521.7)
¹H-NMR ([D₆]-DMSO): δ (ppm) = 0.85 (t, J = 7 Hz, 3H, CH₃), 1.13-1.34 (m, 16H, (CH₂)₈), 1.61 (quint, J = 7 Hz, 2H, CH₂CH₂CO), 2.88 (t, J = 7 Hz, 2H, CH₂CH₂CO), 3.47 (s, 2H, CH₂COOH), 4.23 (t, J = 5 Hz, 2H, NCH₂CH₂O), 4.80 (t, J = 5 Hz, 2H, NCH₂CH₂O), 6.71 (d, J = 8 Hz, 1H, aromat. H), 6.72 (s, 1H, aromat. H), 6.79 (d, J = 8 Hz, 1H, aromat. H), 7.16 (t, J = 8 Hz, 1H, aromat. H), 7.25 (t, J = 8 Hz, 1H, aromat. H), 7.36 (t, J = 8 Hz, 1H, aromat. H), 7.74 (d, J = 8 Hz, 1H, aromat. H), 7.93 (d, J = 8 Hz, 1H, aromat. H)

### Beispiel 14

### 1-{2-[4-(Carboxymethyl)phenoxy]ethyl}-3-dodecanoylindol-2-carbonsäure

Darstellung gemäß Beispiel 12 unter Verwendung von 207 mg (0.72 mmol) 2-[4-(2-Bromethoxy)phenyl]essigsäureethylester anstelle von 2-[4-(Brommethyl)phenoxy]essigsäureethylester. Das Produkt wird aus Petrolether ausgefällt.
Ausbeute: 63 mg (20 %)
Schmp.: 130-131°C
C₃₁H₃₉NO₆ (521.7)
¹H-NMR ([D₆]-DMSO): δ (ppm) = 0.85 (t, J = 7 Hz, 3H, CH₃), 1.13-1.37 (m, 16H, (CH₂)₈), 1.62 (quint, J = 7 Hz, 2H, CH₂CH₂CO), 2.88 (t, J = 7 Hz, 2H, CH₂CH₂CO), 3.43 (s, 2H, CH₂COOH), 4.23 (t, J = 5 Hz, 2H, NCH₂CH₂O), 4.80 (t, J = 5 Hz, 2H, NCH₂CH₂O), 6 .76 (d, J = 8 Hz, 2H, aromat. H), 7.10 (d, J = 8 Hz, 2H, aromat. H), 7.24 (t, J = 8 Hz, 1H, aromat. H), 7.36 (t, J = 8 Hz, 1H, aromat. H), 7.72 (d, J = 8 Hz, 1H, aromat. H), 7.92 (d, J = 8 Hz, 1H, aromat. H)

### Beispiel 15

Die Wirksamkeit der erfindungsgemäßen Verbindungen läßt sich anhand der Hemmung der Phospholipase A₂ bestimmen. Die verwendete Testmethode wurde bereits beschrieben (s. Lehr M., In-vitro assay for the evaluation of phospholipase A₂ inhibitors using bovine platelets and HPLC with UV-detection. Pharm.Pharmacol.Lett. 1992, 2, 176-179). Die Testsubstanzen wurden üblicherweise in DMSO gelöst.
Die bei der Testung erfindungsgemäßer Verbindungen erhaltenen Ergebnisse sind in der nachfolgenden Tabelle 5 aufgeführt. Für die bereits bekannten PLA₂-Inhibitoren (S)-N-Hexadecylpyrrolidin-2-carboxamid (McGregor et al., US Patent 4792555), 1-Methyl-3-octadecanoylindol-2-carbonsäure und 3-(1,3,5-Trimethyl-4-octadecanoylpyrrol-2-yl)propionsäure (Lehr M., WO95/13266) wurden mit dem verwendeten Testsystem die in Tabelle 6 angegebenen Hemmwerte erhalten.

**Tabelle 5:**

| Verbindung von Beispiel Nr. | Hemmung der cytosolischen PLA₂ IC₅₀ [µM] |
|---|---|
| 2 | 4.6 |
| 3 | 3.0 |
| 13 | 1.6 |
| 14 | 1.6 |

**Tabelle 6:**

| Verbindung | Hemmung der cytosolischen PLA₂ IC₅₀ [µM] |
|---|---|
| (S)-N-Hexadecyl-2-pyrrolidincarboxamid | 13 |
| | |
| 3-(1,3,5-Trimethyl-4-octadecanoylpyrrol-2-yl)propionsäure | 13 |
| | |
| 1-Methyl-3-octadecanoylindol-2-carbonsäure | 8 |

## Patentansprüche

1. Substituierte Pyrrolverbindungen und substituierte Indolverbindungen der allgemeinen Formeln I und II: 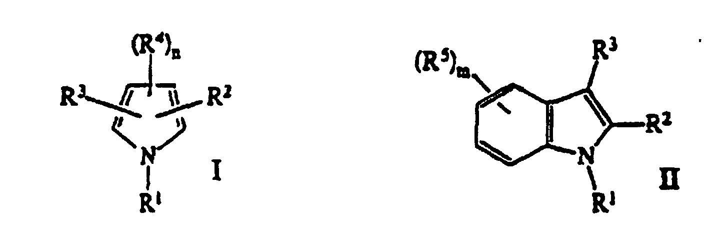 worin
R¹ ein Rest -Y¹-Ar-Y²-Y³ ist, wobei Y¹ ein C₁-C₁₂-Alkyl-, C₂-C₁₂-Alkenyl-, C₁-C₁₂-Alkyloxy- oder C₂-C₁₂-Alkenyloxy-Rest ist, der gegebenenfalls durch ein oder mehrere Sauerstoffatome unterbrochen sein kann, Ar eine Arylgruppe ist, die gegebenenfalls mit 1 bis 3 Substituenten ausgewählt aus der Gruppe R⁶, R⁷ und R⁸ substituiert sein kann, Y² ein C₁-C₁₂-Alkyl-, C₂-C₁₂-Alkenyl-, C₁-C₁₂-Alkyloxy- oder C₂-C₁₂-Alkenyloxy-Rest ist, der gegebenenfalls durch ein oder mehrere Sauerstoffatome unterbrochen sein kann, und Y³ für - COOR¹⁷, -CONR¹⁷R¹⁷, -CONHCOR¹⁹, -CONHS(O)₂R¹⁹, - CONHNHS(O)₂R¹⁹ bzw. -Tz steht, wobei Tz 1H- oder 2H-Tetrazol-5-yl bedeutet;
R² für -COOR¹⁷, -Y⁴-COOR¹⁷, -CONR¹⁷R¹⁷, -Y⁴-CONR¹⁷R¹⁷, - CONHCOR¹⁹, -Y⁴-CONHCOR¹⁹, -CONHS(O)₂R¹⁹, -Y⁴-CONHS(O)₂R¹⁹, -CONHNHS(O)₂R¹⁹, -Y⁴-CONHNHS(O)₂R¹⁹, -Tz bzw. -Y⁴-Tz steht, wobei Y⁴ eine C₁-C₈-Alkyl- oder C₂-C₈-Alkenylgruppe ist, die gegebenenfalls durch ein Sauerstoffatom unterbrochen sein kann und Tz 1H- oder 2H-Tetrazol-5-yl bedeutet;
R³ für -CO-R⁹ steht, wobei R⁹ für -Y⁵, -Aryl oder -Y⁵-Aryl steht, wobei Y⁵ eine C₁-C₁₉-Alkyl- oder C₂-C₁₉-Alkenyl- oder -Alkinyl-Gruppe ist, die gegebenenfalls durch ein oder mehrere Sauerstoffatome unterbrochen sein kann, und Aryl eine gegebenenfalls mit 1 bis 3 Substituenten ausgewählt aus der Gruppe R¹⁰, R¹¹ und R¹² substituierte Arylgruppe ist;
jeder Rest R⁴ unabhängig voneinander für ein Wasserstoffatom, ein Halogenatom, -CF₃, -Y⁶, -Aryl oder -Y⁶-Aryl steht, wobei Y⁶ eine C₁-C₈-Alkyl- oder C₂-C₈-Alkenyl- oder -Alkinyl-Gruppe ist, die gegebenenfalls durch ein oder mehrere Sauerstoffatome unterbrochen sein kann, und Aryl eine gegebenenfalls mit 1 bis 3 Substituenten ausgewählt aus der Gruppe R¹³, R¹⁴ und R¹⁵ substituierte Arylgruppe ist und n die Zahl 2 ist; und wobei zwei Reste Y⁶, sofern es sich um zwei benachbart stehende Alkylreste handelt, zusammen mit dem Kohlenstoffatom, an das sie gebunden sind, einen 5-8-gliedrigen Ring bilden können, der gegebenenfalls mit 1 bis 2 C₁-C₄-Alkylgruppen substituiert sein kann;
jeder Rest R⁵ unabhängig voneinander für ein Wasserstoffatom oder R¹⁶ steht und m die Zahl 4 ist;
R⁶, R⁷, R⁸, R¹⁰, R¹¹, R¹², R¹³, R¹⁴, R¹⁵ und R¹⁶ unabhängig voneinander ausgewählt sind aus:
(1) C₁-C₂₀-Alkylgruppe, die gegebenenfalls durch ein Sauerstoffheteroatom unterbrochen sein kann;
(2) C₂₋₂₀-Alkenylgruppe, die gegebenenfalls durch ein Sauerstoffheteroatom unterbrochen sein kann;
(3) C₂₋₂₀-Alkinylgruppe, die gegebenenfalls durch ein Sauerstoffheteroatom unterbrochen sein kann;
(4) Halogen;
(5) -CF₃;
(6) Perhalo-C₁-C₆-Alkenyl;
(7) -CN;
(8) -NO₂;
(9) -OR¹⁷;
(10) -SR¹⁷;
(11) -COOR¹⁷;
(12) -COR¹⁸;
(13) -COCH₂OH;
(14) -NHCOR¹⁷;
(15) -NR¹⁷R¹⁷;
(16) -NHS(O)₂R¹⁷;
(17) -SOR¹⁷;
(18) -S(O)₂R¹⁷;
(19) -CONR¹⁷R¹⁷;
(20) -SO₂NR¹⁷R¹⁷;
(21) -OOCR¹⁸;
(22) -OOCNR¹⁷R¹⁷;
(23) -OOCOR¹⁷;
(24) -(CH₂)ᵣOR²³;
(25) -(CH₂)ᵣSR²³;
(26) -(CH₂)ᵣNHR²³;
(27) -(CH₂)ₛR²⁰;
R¹⁷ jeweils unabhängig voneinander Wasserstoff, eine C₁₋C₂₀-Alkyl-, C₂₋C₁₉-Alkenyl- oder -Alkinyl-Gruppe, die gegebenenfalls durch ein Sauerstoffheteroatom unterbrochen sein kann, oder -(CH₂)ₜR²⁰ bedeutet;
R¹⁸ jeweils unabhängig voneinander R¹⁷, -CF₃, -(CH₂)ᵤCOOH oder -(CH₂)ᵤCOOR²¹ bedeutet;
R¹⁹ jeweils unabhängig voneinander R¹⁷ oder -CF₃ bedeutet;
R²⁰ jeweils unabhängig voneinander Aryl, substituiert mit einer oder zwei R²²-Gruppen bedeutet;
R²¹ jeweils unabhängig voneinander C₁₋C₆-Alkyl, Benzyl oder Phenyl bedeutet;
R²² jeweils unabhängig voneinander Wasserstoff, Halogen, C₁₋C₁₂-Alkyl, C₁₋C₁₂-Alkoxy, C₁₋C₁₂-Alkylthio, C₁₋C₁₂-Alkylsulfonyl, C₁₋C₁₂-Alkylcarbonyl, -CF₃, -CN oder -NO₂ bedeutet;
R²³ jeweils unabhängig voneinander Wasserstoff oder -COR²¹ bedeutet;
r 1 bis 20 ist;
s und t jeweils unabhängig voneinander 0 bis 12 sind;
u 0 bis 4 ist;
sowie deren pharmazeutisch verträglichen Salze und Ester.

2. Verbindung nach Anspruch 1 der allgemeinen Formel I': 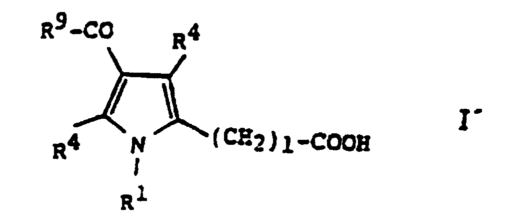 worin die Reste R⁴ jeweils unabhängig voneinander ausgewählt sind aus einem Wasserstoffatom, einer C₁₋₅-Alkyl-, Benzyl-, oder Phenylgruppe; und 1 eine ganze Zahl von 0 bis 3 ist.

3. Verbindung nach Anspruch 2, worin R⁹ für eine (C₇₋₁₇-Alkyl) oder (Aryl-C₁₋₁₇-alkyl)gruppe steht.

4. Verbindung nach Anspruch 2, worin die Reste R⁴ jeweils unabhängig voneinander ausgewählt sind aus einem Wasserstoffatom, einer Methyl-, Neopentyl-, Phenyl- und Benzylgruppe.

5. Verbindung nach Anspruch 2, worin 1 den Wert 1 oder 2 hat, R⁹ eine (C₇₋₁₇-Alkyl)- oder (Aryl-C₁₋₁₇-alkyl)gruppe ist und die Reste R⁴ jeweils unabhängig voneinander ein Wasserstoffatom oder eine Methyl- oder Benzylgruppe sind.

6. Verbindung nach Anspruch 5, worin 1 den Wert 1 oder 2 hat, R⁹ eine (C₇₋₁₇-Alkyl)gruppe und die Reste R⁴ jeweils eine Methylgruppe sind.

7. Verbindung nach Anspruch 5, worin der Rest R⁹-CO- eine Dodecanoylgruppe ist und die Reste R⁴ jeweils unabhängig voneinander ein Wasserstoffatom oder eine Methyl- oder Benzylgruppe sind.

8. Verbindung nach einem der Ansprüche 1 bis 7, worin Y¹ eine Methylen- oder Ethylengruppe ist, Ar eine Phenylengruppe ist und Y² eine Ethenylen- oder Ethylengruppe in meta- oder para-Stellung zu Y¹ darstellt.

9. Verbindung nach Anspruch 1, nämlich 1-[4-(2-Carboxyethyl)benzyl)-4-dodecanoylpyrrol-2-carbonsäure, (E)-3-{[2-(Carboxymethyl)-4-dodecanoyl-3,5-dimethylpyrrol-1-yl]methyl}zimtsäure, 3-(3-{[2-(Carboxymethyl)-4-dodecanoyl-3,5-dimethylpyrrol-1-yl]methyl}phenyl)propionsäure, (E)-4-{[2-(Carboxymethyl)-4-dodecanoyl-3,5-dimethylpyrrol-1-yl]methyl}zimtsäure, 3-(4-{[2-(Carboxymethyl)-4-dodecanoyl-3,5-dimethylpyrrol-1-yl]methyl} phenyl)propionsäure, (E)-4-{[2-(2-Carboxyethyl)-3,5-dimethyl-4-octadecanoylpyrrol-1-yl]methyl}zimtsäure, 3-(4-{[2-(2-Carboxyethyl)-3,5-dimethyl-4-octadecanoylpyrrol-1-yl]methyl}phenyl)propionsäure.

10. Verbindung nach Anspruch 1 der allgemeinen Formel II' 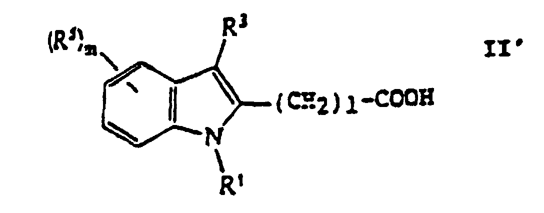 worin l eine ganze Zahl von 0 bis 3 ist.

11. Verbindung nach Anspruch 10, worin R³ eine (C₇₋₁₇-Alkyl)-CO- oder (Aryl-C₁₋₁₇-Alkyl)-CO-gruppe, insbesondere eine Dodecanoylgruppe ist.

12. Verbindung nach Anspruch 10 oder 11, worin l die Zahl 0 ist.

13. Verbindung nach einem der Ansprüche 10 bis 12, worin drei Reste R⁵ ein Wasserstoffatom darstellen und der andere Rest R⁵ ein Wasserstoffatom, eine 4-Chlor-, 5-Chlor- oder 5-Methoxygruppe ist.

14. Verbindung nach einem der Ansprüche 10 bis 13, worin der Rest Y¹ des Restes R¹ eine Methylen- oder Ethoxygruppe ist, Ar eine Phenylengruppe ist und Y² eine Methylen-, Ethylen-, Ethenylen-, oder eine Methoxygruppe ist.

15. Verbindung nach Anspruch 10, nämlich (E)-1-[3-(2-Carboxyethen-1-yl)benzyl]-3-dodecanoylindol-2-carbonsäure, 1-[3-(2-Carboxyethyl)benzyl]-3-dodecanoylindol-2-carbonsäure, (E)-1-[4-(2-Carboxyethen-1-yl)benzyl]-3-dodecanoylindol-2-carbonsäure, 1-[4-(2-Carboxyethyl) benzyl]-3-dodecanoylindol-2-carbonsäure, 1-[4-(Carboxymethoxy)benzyl]-3-dodecanoylindol-2-carbonsäure, 1-{2-[3-(Carboxymethyl)phenoxy]ethyl}-3-dodecanoylindol-2-carbonsäure, 1-{2-[4-(Carboxymethyl)phenoxy]ethyl}-3-dodecanoylindol-2-carbonsäure.

16. Arzneimittel, enthaltend zumindest eine Verbindung nach einem der Ansprüche 1 bis 15, gegebenenfalls zusammen mit üblichen pharmazeutisch verträglichen Hilfsstoffen und/oder Zusatzstoffen.

17. Arzneimittel nach Anspruch 16 zur Verwendung als Hemmstoff der Phospholipase A₂.

18. Verwendung von zumindest einer Verbindung nach einem der Ansprüche 1 bis 15 zur Herstellung eines Arzneimittels zur Prävention und/oder zur Behandlung von Erkrankungen, die durch eine erhöhte Aktivität der Phospholipase A₂ verursacht bzw. mitverursacht werden, wobei es sich bei den Erkrankungen insbesondere um Entzündungen, Schmerz, Fieber, Allergien, Asthma, Psoriasis und Endotoxinschock handelt.

19. Verfahren zur Herstellung eines Arzneimittels nach einem der Ansprüche 16 und 17, **dadurch gekennzeichnet, daß** man zumindest eine Verbindung nach einem der Ansprüche 1 bis 15 in einer zur Verabreichung geeigneten Form bereitstellt, gegebenenfalls unter Verwendung üblicher pharmazeutischer Träger und/oder Zusatzstoffe.

20. Verfahren zur Herstellung einer substituierten Pyrrolverbindung und substituierten Indolverbindung nach Anspruch 1, bei dem eine Pyrrolverbindung bzw. eine Indolverbindung der Formel 3'bzw. 4' 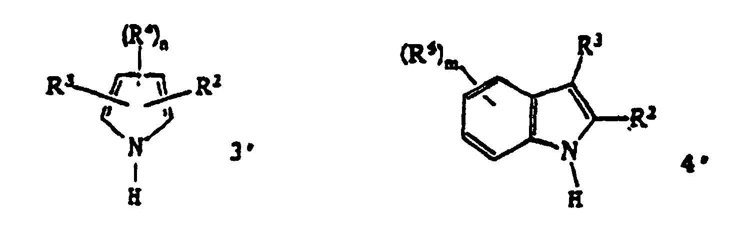 auf übliche Art und Weise unter Verwendung einer Base mit dem entsprechenden Alkylhalogenid Hal-Y¹-aryl-Y²-Y³ alkyliert wird, wobei die Reste R², R³, R⁴, R⁵, Y¹, Y², Y³ und Aryl sowie m und n wie in Anspruch 1 definiert sind und Hal ein Halogenatom, insbesondere ein Bromatom darstellt.

## Claims

1. Substituted pyrrole compounds and substituted indole compounds of the general formulae I and II: 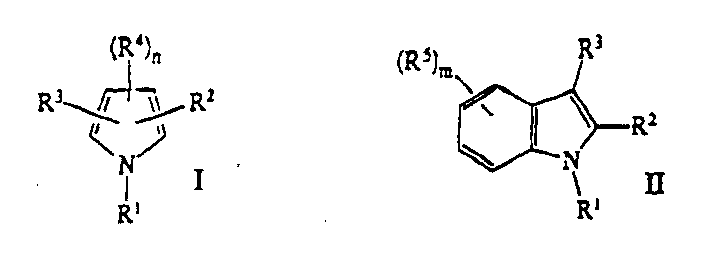 in which
R¹ is a radical -Y¹-Ar-Y²-Y³ where Y¹ is a C₁-C₁₂-alkyl, C₂-C₁₂-alkenyl, C₁-C₁₂-alkyloxy or C₂-C₁₂-alkenyloxy radical which can optionally be interrupted by one or more oxygen atoms, Ar is an aryl group which may optionally be substituted by 1 to 3 substituents selected from the group R⁶, R⁷ and R⁸, Y² is a C₁-C₁₂-alkyl, C₂-C₁₂-alkenyl, C₁-C₁₂-alkyloxy or C₂-C₁₂-alkenyloxy radical which can optionally be interrupted by one or more oxygen atoms, and Y³ is -COOR¹⁷, -CONR¹⁷R¹⁷, - CONHCOR¹⁹, -CONHS(O)₂R¹⁹, -CONHNHS(O)₂R¹⁹, or -Tz where Tz is 1H- or 2H-tetrazol-5-yl;
R² is -COOR¹⁷, -Y⁴-COOR¹⁷, -CONR¹⁷R¹⁷, -Y⁴-CONR¹⁷R¹⁷, - CONHCOR¹⁹, -Y⁴-CONHCOR¹⁹, -CONHS(O)₂R¹⁹, -Y⁴-CONHS(O)₂R¹⁹, -CONHNHS(O)₂R¹⁹, -Y⁴-CONHNHS(O)₂R¹⁹, -Tz or -Y⁴-Tz where Y⁴ is a C₁-C₈-alkyl or C₂-C₈-alkenyl group which can optionally be interrupted by an oxygen atom, and Tz is 1H- or 2H-tetrazol-5-yl;
R³ is -CO-R⁹ where R⁹ is -Y⁵, -aryl or -Y⁵-aryl, where Y⁵ is a C₁-C₁₉-alkyl or C₂-C₁₉-alkenyl or -alkynyl group which can optionally be interrupted by one or more oxygen atoms, and aryl is an aryl group which is optionally substituted by 1 to 3 substituents selected from the group of R¹⁰, R¹¹ and R¹²;
each R⁴ radical is, independently of the others, a hydrogen atom, a halogen atom, -CF₃, -Y⁶, -aryl or -Y⁶-aryl, where Y⁶ is a C₁-C₈-alkyl or C₂-C₈-alkenyl or -alkynyl group which can optionally be interrupted by one or more oxygen atoms, and aryl is an aryl group which is optionally substituted by 1 to 3 substituents selected from the group of R¹³, R¹⁴ and R¹⁵, and n is the number 2; and where two Y⁶ radicals can, if they are two adjacent alkyl radicals, form together with the carbon atom to which they are bonded a 5-8-membered ring which may optionally be substituted by 1 to 2 C₁-C₄-alkyl groups;
each R⁵ radical is, independently of the others, a hydrogen atom or R¹⁶, and m is the number 4;
R⁶, R⁷, R⁸, R¹⁰, R¹¹, R¹², R¹³, R¹⁴, R¹⁵ and R¹⁶ are selected independently of one another from:
(1) C₁-C₂₀-alkyl group which can optionally be interrupted by an oxygen heteroatom;
(2) C₂-C₂₀-alkenyl group which can optionally be interrupted by an oxygen heteroatom;
(3) C₂-C₂₀-alkynyl group which can optionally be interrupted by an oxygen heteroatom;
(4) halogen;
(5) -CF₃;
(6) perhalo-C₁-C₆-alkenyl;
(7) -CN;
(8) -NO₂;
(9) -OR¹⁷;
(10) -SR¹⁷;
(11) -COOR¹⁷;
(12) -COR¹⁸;
(13) -COCH₂OH;
(14) -NHCOR¹⁷;
(15) -NR¹⁷R¹⁷;
(16) -NHS(O)₂R¹⁷;
(17) -SOR¹⁷;
(18) -S(O)₂R¹⁷;
(19) -CONR¹⁷R¹⁷;
(20) -SO₂NR¹⁷R¹⁷;
(21) -OOCR¹⁸;
(22) -OOCNR¹⁷R¹⁷;
(23) -OOCOR¹⁷;
(24) - (CH₂)ᵣOR²³;
(25) - (CH₂)ᵣSR²³;
(26) -(CH₂)ᵣNHR²³;
(27) -(CH₂)ₛR²⁰;
R¹⁷ is in each case, independently of one another, hydrogen, a C₁-C₂₀-alkyl or C₂-C₁₉-alkenyl or -alkynyl group which can optionally be interrupted by an oxygen heteroatom, or - (CH₂)ₜR²⁰;
R¹⁸ is in each case, independently of one another, R¹⁷, - CF₃, - (CH₂)ᵤCOOH or - (CH₂)ᵤCOOR²¹;
R¹⁹ is in each case, independently of one another, R¹⁷ or -CF₃;
R²⁰ is in each case, independently of one another, aryl substituted by one or two R²² groups;
R²¹ is in each case, independently of one another, C₁-C₆-alkyl, benzyl or phenyl;
R²² is in each case, independently of one another, hydrogen, halogen, C₁-C₁₂-alkyl, C₁-C₁₂-alkoxy, C₁-C₁₂-alkylthio, C₁-C₁₂-alkylsulfonyl, C₁-C₁₂-alkylcarbonyl, -CF₃, -CN or -NO₂;
R²³ is in each case, independently of one another, hydrogen or -COR²¹;
r is 1 to 20;
s and t are in each case, independently of one another, 0 to 12;
u is 0 to 4;
and the pharmaceutically suitable salts and esters thereof.

2. A compound as claimed in claim 1 of the general formula I': 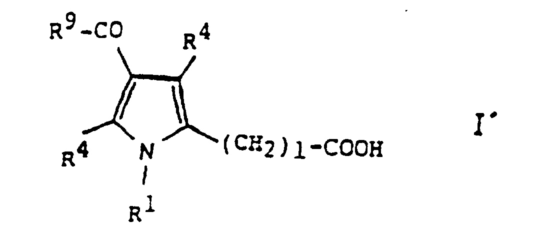 in which the R⁴ radicals are each, independently of one another, selected from a hydrogen atom, a C₁₋₅-alkyl, benzyl or phenyl group; and 1 is an integer from 0 to 3.

3. A compound as claimed in claim 2, in which R⁹ is a (C₇₋₁₇-alkyl) or (aryl-C₁₋₁₇-alkyl) group.

4. A compound as claimed in claim 2, in which the R⁴ radicals are each, independently of one another, selected from a hydrogen atom, a methyl, neopentyl, phenyl and benzyl group.

5. A compound as claimed in claim 2, in which 1 has the value 1 or 2, R⁹ is a (C₇₋₁₇-alkyl) or (aryl-C₁₋₁₇-alkyl) group, and the R⁴ radicals are each, independently of one another, a hydrogen atom or a methyl or benzyl group.

6. A compound as claimed in claim 5, in which 1 has the value 1 or 2, R⁹ is a (C₇₋₁₇-alkyl) group and the R⁴ radicals are each a methyl group.

7. A compound as claimed in claim 5, in which the R⁹-CO- radical is a dodecanoyl group, and the R⁴ radicals are each, independently of one another, a hydrogen atom or a methyl or benzyl group.

8. A compound as claimed in any of claims 1 to 7, in which Y¹ is a methylene or ethylene group, Ar is a phenylene group, and Y² is an ethenylene or ethylene group in the position meta or para to Y¹.

9. A compound as claimed in claim 1, namely 1-[4-(2-carboxyethyl)benzyl]-4-dodecanoylpyrrole-2-carboxylic acid, (E)-3-{[2-(carboxymethyl)-4-dodecanoyl-3,5-dimethylpyrrol-1-yl]methyl}cinnamic acid, 3-(3-{[2-(carboxymethyl)-4-dodecanoyl-3,5-dimethylpyrrol-1-yl]methyl}phenyl)propionic acid, (E)-4-{[2-(carboxymethyl)-4-dodecanoyl-3,5-dimethylpyrrol-1-yl]methyl}cinnamic acid, 3-(4-{[2-(carboxymethyl)-4-dodecanoyl-3,5-dimethylpyrrol-1-yl]methyl}phenyl)propionic acid, (E)-4-{[2-(2-carboxyethyl)-3,5-dimethyl-4-octadecanoylpyrrol-1-yl]methyl}cinnamic acid, 3-(4-{[2-(2-carboxyethyl)-3,5-dimethyl-4-octadecanoylpyrrol-1-yl]methyl}phenyl)propionic acid.

10. A compound as claimed in Claim 1 of the general formula II' 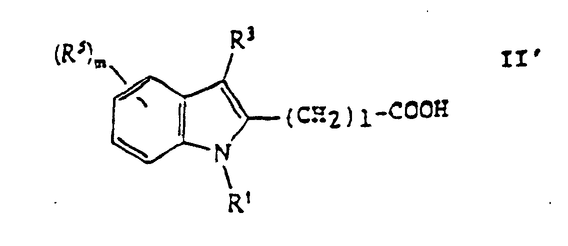 in which 1 is an integer from 0 to 3.

11. A compound as claimed in claim 10, in which R³ is a (C₇₋₁₇-alkyl)-CO- or (aryl-C₁₋₁₇-alkyl)-CO- group, in particular a dodecanoyl group.

12. A compound as claimed in claim 10 or 11, in which 1 is the number 0.

13. A compound as claimed in any of claims 10 to 12, in which three R⁵ radicals are a hydrogen atom, and the other R⁵ radical is a hydrogen atom, a 4-chloro 5-chloro or 5-methoxy group.

14. A compound as claimed in any of claims 10 to 13, in which the Y¹ radical of the R¹ radical is a methylene or ethoxy group, Ar is a phenylene group, and Y² is a methylene, ethylene, ethenylene or a methoxy group.

15. A compound as claimed in Claim 10, namely (E)-1-[3-(2-carboxyethen-1-yl)benzyl]-3-dodecanoylindole-2-carboxylic acid, 1-[3-(2-carboxyethyl)benzyl]-3-dodecanoylindole-2-carboxylic acid, (E-1-[4-(2-carboxyethen-1-yl)benzyl)-3-dodecanoylindole-2-carboxylic acid, 1-[4-(2-carboxyethyl)benzyl]-3-dodecanoylindole-2-carboxylic acid, 1-[4-(carboxymethoxy)benzyl]-3-dodecanoylindole-2-carboxylic acid, 1-{2-[3-(carboxymethyl)phenoxy]ethyl}-3-dodecanoylindole-2-carboxylic acid, 1-{2-[4-(carboxymethyl)-phenoxy]ethyl}-3-dodecanoylindole-2-carboxylic acid.

16. A pharmaceutical comprising at least one compound as claimed in any of claims 1 to 15, where appropriate together with conventional pharmaceutically suitable ancillary substances and/or additives.

17. A pharmaceutical as claimed in claim 16 for use as inhibitor of phospholipase A₂.

18. The use of at least one compound as claimed in any of claims 1 to 15 for producing a pharmaceutical for preventing and/or treating disorders caused or partly caused by an increased activity of phospholipase A₂, the disorders being, in particular, inflammations, pain, fever, allergies, asthma, psoriasis and endotoxic shock.

19. A process for producing a pharmaceutical as claimed in either of claims 16 and 17, which comprises producing a form suitable for administration from at least one compound as claimed in any of claims 1 to 15, where appropriate using conventional pharmaceutical excipients and/or additives.

20. A process for preparing a substituted pyrrole compound and substituted indole compound as claimed in claim 1, in which a pyrrole compound or an indole compound of the formula 3' or 4' 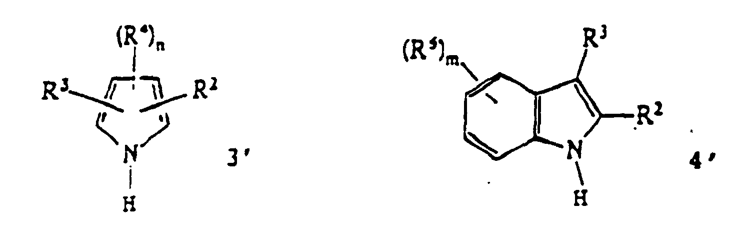 is alkylated in a conventional way with the appropriate alkyl halide Hal-Y¹-aryl-Y²-Y³, using a base, where the radicals R², R³, R⁴, R⁵, Y¹, Y², Y³ and aryl, and m and n are as defined in claim 1, and Hal is a halogen atom, in particular a bromine atom.

## Revendications

1. Composés substitués de pyrrole et composés substitués d'indole des formules générales I et II: 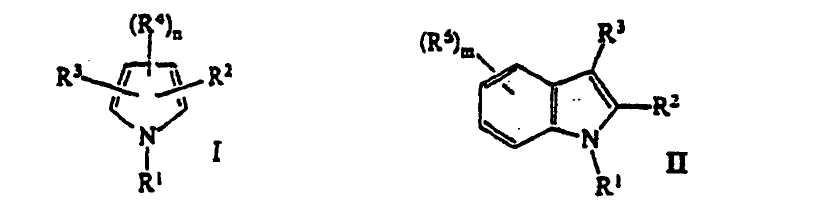 dans lesquelles
R¹ représente un reste -Y¹-Ar-Y²-Y³, où Y¹ représente un radical alkyle en C₁ à C₁₂, alcényle en C₂ à C₁₂, alkyloxy en C₁ à C₁₂ ou alcényloxy en C₂ à C₁₂, qui peut éventuellement être interrompu par un ou plusieurs atomes d'oxygène, Ar est un groupe aryle, qui peut éventuellement être substitué par 1 à 3 substituants choisis dans le groupe formé par R⁶, R⁷ et R⁸, Y² représente un radical alkyle en C₁ à C₁₂, alcényle en C₂ à C₁₂, alkyloxy en C₁ à C₁₂ ou alcényloxy en C₂ à C₁₂, qui peut éventuellement être interrompu par un ou plusieurs atomes d'oxygène, et Y³ représente - COOR¹⁷, -CONR¹⁷R¹⁷, -CONHCOR¹⁹, -CONHS(O)₂R¹⁹, - CONHNHS(O)₂R¹⁹, ou -Tz, où Tz représente du 1H- ou du 2H-tétrazol-5-yle,
R² représente -COOR¹⁷, -Y⁴- -COOR¹⁷, -CONR¹⁷R¹⁷, -Y⁴- -CONR¹⁷R¹⁷, - CONHCOR¹⁹, -Y⁴- -CONHCOR¹⁹, -CONHS(O)₂R¹⁹, -Y⁴-CONHS(O)₂R¹⁹, - CONHNHS(O)₂R¹⁹, -Y⁴- CONHNHS(O)₂R¹⁹, -Tz ou -Y⁴-Tz, où Y⁴ représente un radical alkyle en C₁ à C₈ ou alcényle en C₂ à C₈ et où Tz représente du 1 H- ou du 2H-tétrazol-5-yle,
R³ représente -CO-R⁹, où R⁹ représente -Y⁵, -aryle ou -Y⁵_aryle, où Y⁵ est un groupe alkyle en C₁ à C₁₉ ou alcényle ou alcynyle en C₂ à C₁₉, qui peut éventuellement être interrompu par un ou plusieurs atomes d'oxygène, et aryle représente un groupe aryle éventuellement substitué par 1 à 3 substituants choisis dans le groupe formé par R¹⁰, R¹¹ et R¹²,
chacun des restes R⁴ représente indépendamment des autres un atome d'hydrogène, un atome d'halogène, -CF₃, -Y⁶, -aryle ou -Y⁶-aryle, où Y⁶ représente un radical alkyle en C₁ à C₈ ou alcényle ou alcynyle en C₂ à C₈, qui peut éventuellement être interrompu par un ou plusieurs atomes d'oxygène, et aryle représente un groupe aryle éventuellement substitué par 1 à 3 substituants choisis dans le groupe formé par R¹³, R¹⁴ et R¹⁵ et n est le nombre 2, et où deux restes Y⁶, dans la mesure où il s'agit de deux radicaux alkyle voisins, peuvent former ensemble avec l'atome de carbone auquel ils sont liés un noyau pentagonal à octogonal, qui peut éventuellement être substitué par 1 à 2 radicaux alkyle en C₁ à C₄,
chacun des restes R⁵ représente indépendamment des autres un atome d'hydrogène ou R¹⁶ et m est le nombre 4,
R⁶, R⁷, R⁸, R¹⁰, R¹¹, R¹², R¹³, R¹⁴, R¹⁵ et R¹⁶ sont choisis indépendamment les uns des autres parmi:
(1) des radicaux alkyle en C₁ à C₂₀, qui peuvent éventuellement être interrompus par un hétéroatome d'oxygène,
(2) des radicaux alcényle en C₂ à C₂₀, qui peuvent éventuellement être interrompus par un hétéroatome d'oxygène,
(3) des radicaux alcynyle en C₂ à C₂₀, qui peuvent éventuellement être interrompus par un hétéroatome d'oxygène,
(4) un halogène,
(5) -CF₃,
(6) un groupe perhalo-(C₁-C₆)-alcényle,
(7) -CN,
(8) -NO₂,
(9) -OR¹⁷,
(10) -SR¹⁷,
(11) -COOR¹⁷,
(12) -COR¹⁸,
(13) -COCH₂OH,
(14) -NHCOR¹⁷,
(15) -NR¹⁷R^{17,}
(16) -NHS(O)₂R¹⁷,
(17) -SOR¹⁷,
(18) -S(O)₂R¹⁷,
(19) -CONR¹⁷R¹⁷,
(20) -SO₂NR¹⁷R¹⁷,
(21) -OOCR¹⁸,
(22) -OOCNR¹⁷R¹⁷,
(23) -OOCOR¹⁷,
(24) -(CH₂)ᵣOR²³,
(25) -(CH₂)ᵣSR²³,
(26) -(CH₂)ᵣNHR^{23,}
(27) -(CH₂)ᵣR²⁰,
les R¹⁷ représentent à chaque fois indépendamment l'un de l'autre de l'hydrogène, un radical alkyle en C₁ à C₂₀ ou bien alcényle ou alcynyle en C₂ à C₁₉, qui peut éventuellement être interrompu par un hétéroatome d'oxygène,
les R¹⁸ représentent à chaque fois indépendamment l'un de l'autre R¹⁷, -CF₃, -(CH₂)ᵤCOOH ou -(CH₂)ᵤCOOR²¹,
les R¹⁹ représentent à chaque fois indépendamment l'un de l'autre R¹⁷ ou - CF₃,
les R²⁰ représentent à chaque fois indépendamment l'un de l'autre un radical aryle, substitué part un ou deux groupes R²²,
les R²¹ représentent à chaque fois indépendamment l'un de l'autre un radical alkyle en C₁ à C₆, benzyle ou phényle,
les R²² représentent à chaque fois indépendamment l'un de l'autre de l'hydrogène, un halogène, ou un radical alkyle en C₁ à C₁₂, alcoxy en C₁ à C₁₂, alkylthio en C₁ à C₁₂, alkylsulfonyle en C₁ à C₁₂, alkylcarbonyle en C₁ à C₁₂, -CF₃, -CN ou -NO₂,
les R²³ représentent à chaque fois indépendamment l'un de l'autre de l'hydrogène ou -COR²¹,
r a une valeur de 1 à 20,
s et t ont à chaque fois indépendamment l'un de l'autre une valeur de 0 à 12,
u a une valeur de 0 à 4,
ainsi que leurs sels et esters pharmaceutiquement compatibles.

2. Composé selon la revendication 1, de la formule générale l': 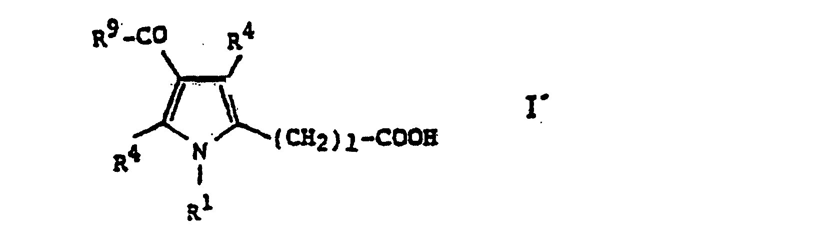 dans laquelle les restes R⁴ sont choisis, à chaque fois indépendamment l'un de l'autre, parmi un atome d'hydrogène, un radical alkyle en C₁ à C₅, benzyle ou phényle, et I est un nombre entier de 0 à 3.

3. Composé selon la revendication 2, dans lequel R⁹ représente de préférence un radical alkyle en C₇ à C₁₇ ou aryl(C₁-₁₇)alkyle.

4. Composé selon la revendication 2, dans lequel les restes R⁴ sont à chaque fois choisis indépendamment l'un de l'autre parmi un atome d'hydrogène ou un groupe méthyl, néopentyle, phényle ou benzyle.

5. Composé selon la revendication 2, dans lequel I a la valeur 1 ou 2, R⁹ représente de préférence un radical alkyle en C₇ à C₁₇ ou aryl(C₁₋₁₇)alkyle et les restes R⁴ représentent à chaque fois indépendamment l'un de l'autre un atome d'hydrogène ou un groupe méthyle ou benzyle.

6. Composé selon la revendication 5, dans lequel I a la valeur 1 ou 2 et R⁹ est un groupe alkyle en C₇ à C₁₇ et les restes R⁴ sont à chaque fois un groupe méthyle.

7. Composé selon la revendication 5, dans lequel le reste R⁹-CO- est un groupe dodécanoyle est les restes R⁴ sont à chaque fois indépendamment l'un de l'autre un atome d'hydrogène ou un groupe méthyle ou benzyle.

8. Composé selon l'une quelconque des revendications 1 à 7, dans lequel Y¹ est un groupe méthylène ou éthylène, Ar est un groupe phénylène et Y² est un groupe acétylène ou éthylène en position méta ou para par rapport à Y¹.

9. Composé selon la revendication 1, à savoir de l'acide 1-[4-(2-carboxyéthyl)benzyl]-4-dodécanoylpyrrol-2-carboxylique, de l'acide (E)-3-{[2-(carboxyméthyl)-4-dodécanoyl-3,5-diméthyl-pyrrol-1-yl)méthyl}-cinnamique, de l'acide 3-(3-{[2-(carboxyméthyl)-4-dodécanoyl-3,5-diméthylpyrrol-1-yl)méthyl}phényl)propionique, de l'acide (E)-4-{[2-(carboxyméthyl)-4-dodécanoyl-3,5-di-méthylpyrrol-1-yl)-méthyl}-cinnamique, de l'acide 3-(4-{[2-(carboxyméthyl)-4-dodécanoyl-3,5-diméthylpyrrol-1-yl]méthyl}phényl)propionique, de l'acide (E)-4-{[2-(2-carboxyéthyl)-3,5-diméthyl-4-octadécanoylpyrrol-1-yl]méthyl}-cinnamique, de l'acide 3-(4-{[2-(2-carboxyéthyl)-3,5-diméthyl-4-octadécanoyl-pyrrol-1-yl]méthyl}phényl)propionique.

10. Composé selon la revendication 1, de la formule générale II': 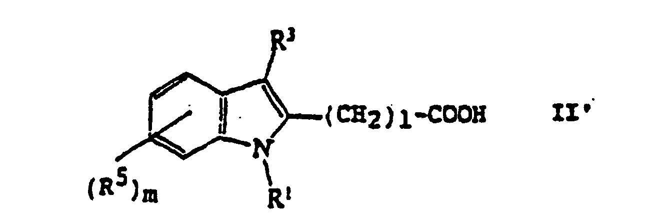 dans laquelle 1 est un nombre entier de 0 à 3.

11. Composé selon la revendication 10, dans lequel R³ représente un groupe (alkyle en C₇ à C₁₇)-CO ou (aryl(C₁₋₁₇)alkyl-CO, en particulier un groupe dodécanoyle.

12. Composé selon la revendication 10 ou 11, dans lequel 1 est le nombre 0.

13. Composé selon l'une quelconque des revendications 10 à 12, dans lequel trois restes R⁵ réprésentent un atome d'hydrogène et les autres restes R⁵ représentent un atome d'hydrogène, un atome de chlore (4), de chlore (5) ou un groupe 5-méthoxy.

14. Composé selon l'une quelconque des revendications 10 à 13, dans lequel le reste Y¹ du reste R¹ représente un groupe méthylène ou éthoxy, Ar est un groupe phénylène et Y² un groupe méthylène, éthylène, acétylène ou méthoxy.

15. Composé selon la revendication 10, à savoir de l'acide (E)-1-(3-(2-carboxyéthén-1-yl)benzyl)-3-dodécanoylindol-2-carboxylique, de l'acide 1-[3-(2-carboxyéthyl)benzyl]-3-dodécanoylindol-2-carboxylique, de l'acide (E)-1-[4-(2-carboxyéthén-1-yl)benzyl]-3-dodécanoylindol-2-carboxylique, de l'acide 1-[4-(2-carboxyéthyl)benzyl]-3-dodécanoylindol-2-carboxylique, de l'acide 1-[4-(carboxyméthoxy)benzyl]-3-dodécanoylindol-2-carboxylique, de l'acide 1-{2-[3-(carboxyméthyl)phénoxy]éthyl}-3-dodécanoylindol-2-carboxylique, de l'acide 1-{2-[4-(carboxyméthyl)phénoxy]éthyl}-3-dodécanoylindol-2-carboxylique.

16. Médicament, contenant au moins un composé selon l'une quelconque des revendications 1 à 15, éventuellement avec des adjuvants et/ou des additifs pharmaceutiquement compatibles.

17. Médicament selon la revendication 15 destiné à être utilisé comme inhibiteur de la phospholipase A2.

18. Utilisation d'au moins un composé selon l'une quelconque des revendications 1 à 15 pour la préparation d'un médicament pour la prévention et/ou le traitement de maladies provoquées individuellement ou de manière concomitante par une activité intensifiée de l'enzyme phospholipase A2, ces maladies étant en particulier des inflammations, des douleurs, de la fièvre, des allergies, de l'asthme, du psoriasis et des chocs endotoxiques.

19. Procédé de préparation d'un médicament selon l'une quelconque des revendications 16 et 17, sous une forme appropriée à l'administration, éventuellement en utilisant des excipients et/ou des additifs pharmaceutiques usuels.

20. Procédé de préparation d'un composé de pyrrole substitué et d'un composé d'indole substitué selon la revendication 1, dans lequel on alkyle un composé de pyrrole ou un composé d'indole de formule 3' ou 4' 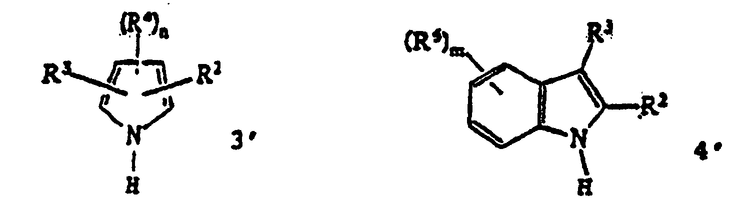 de la manière habituelle en utilisant une base avec l'halogénure d'alkyle correspondant Hal-T¹-aryl-Y²-Y³, les restes R², R³, R⁴, R⁵, Y¹, Y², Y³ et aryle ainsi que m et n étant définis comme dans la revendication 1 et Hal représentant un atome d'halogène, en particulier un atome de brome.
